# EUROPEAN PATENT APPLICATION

(11) **EP 2 336 307 A1**
(43) Date of publication of application: **22.06.2011**
(21) Application number: 09306299.0
(22) Date of filing: 21.12.2009
(51) Int. Cl.: C12N 9/18, C12N 15/82

(54) **Isolated plant GDSL lipase**

(71) Applicant: INSTITUT NATIONAL DE LA RECHERCHE AGRONOMIQUE, 75007 Paris (FR)
(72) Inventor: Bakan, Bénédicte, 44300 Nantes (FR); Rothan, Christophe, 33650 Saint Selve (FR); Chamley, Martine, 33850 Leognan (FR); Marion, Didier, 44300 Nantes (FR); Girard, Marie-Laure, 49800 Brain sur L'Authion (FR); Mounet, Fabien, 33220 Pineuilh (FR)
(74) Representative: Warcoin, Jacques

(57) **Abstract**

The invention relates to an isolated plant GDSL-lipase polypeptide differentially expressed between the different steps of the plant development and/or the different tissues of the plant, a nucleic acid molecule encoding it, and a plant or part of plant genetically engineered to not express it at least partially.

## Description

Described herein are inventions in the field of genetic engineering of plants, including isolated nucleic acid molecules encoding polypeptides to improve agronomic, horticultural, and quality traits. This invention relates generally to nucleic acid sequences encoding proteins that are related to the cutin polymerization in plants. More specifically, the present invention relates to nucleic acid sequences encoding polypeptides being capable of altering the cutin and the use of these sequences in transgenic plants. In particular, the invention is directed to methods for manipulating cuticle polymerization in plants and fruits.

Plant cuticle is a hydrophobic layer that covers all aerial surfaces. Cuticle is a complex hydrophobic assembly characterized by a biopolymer, cutin, coated and filled by waxes. Waxes display a high chemical diversity in the plant kingdom and generally consist of mixtures of aliphatic molecules with very long carbon-chain (up to C60) including alkanes, primary and secondary alcohols, aldehydes, fatty acids, esters as well as cyclic triterpenoids, phenylpropanoids and phenolics (Waltson, 1990).

Cutin, the major component of cuticle, is a polyester of ω and mid-chain hydroxyl C16 and C18 fatty acids and glycerol (Kolattuduky, 1996; Graça et al., 2002). Cutin fulfils multiple functions in plants such as control of non stomatal water loss (Sieber et al., 2000), permeation of gases and solutes (Kersteins, 1996; Riederer and Schreiber, 2001).

Cutin plays also an essential role in the regulation of cell adhesion during plant development by preventing organ fusion as observed in Arabidopsis mutants (Sieber et al., 2000; Nawrath, 2006) or by participating to hull adhesion in grains (Takeda et al., 2008). It is generally admitted that plant cuticles and its polymeric matrix, cutin, are primary barriers to pathogens and that cutin monomers released by fungal cutinase are signalling molecules for both pathogen and plants (Gilbert et al., 1996; Schweizer et al., 1996; Iwamoto et al., 2002).

Cutin monomers, i.e. hydroxy- and epoxy-fatty acids, are synthesized in the epidermal cells from which they are exported and polymerized at the surface of the cells.

Several genes encoding enzymes involved in the biosynthesis of cutin monomers have been identified through Arabidopsis fusion mutants (Wellesen et al., 2001; Schnurr et al., 2004; Xiao et al., 2004; Bessire et al., 2007; Kannangara et al., 2007). The discovery of a transcription factor WIN1 that regulates different genes of cutin biosynthesis has provided a model pathway. This major pathway involved ω-hydroxylases, long-chain acyl-coenzyme A synthetases and glycerol-3-phosphate acyltransferases (Kannangara et al., 2007).

Another pathway, the lipoxygenase-peroxygenase pathway, could be involved in the midchain epoxidation and, coupled with epoxyhydrolases, in the mid-chain hydroxylation of long chain ω-hydroxylated fatty acid precursors (Blee and Schuber, 1993). Glycerol-3-phosphate acyltransferases (GPAT), catalyse the transfer of acyl-CoAs to glycerol-3-phosphate to form lysophosphatidic acid a precursor of acylglycerols. GPAT are also involved in the formation of suberin, a cutin-like polymer that accumulates in roots and in stems (Beisson et al., 2007) and in cutin formation (Li et al., 2007). Therefore monoacylglycerols and lysophosphatidic acids could be candidate precursors for cutin polymerization after translocation in the apoplastic compartment (Li et al., 2007; Pollard et al., 2008). In this regard, it has been demonstrated that, as observed for wax monomers, ATP-binding cassette (ABC) transporters located in the plasmalemma are essential for the exportation of cutin precursors through the plasma membrane from cell into the apoplast (Panikashvili et al., 2007; Bird, 2008).

By contrast, the mechanisms involved in the transport of cutin precursors through the apoplast and its polymerisation are less documented. Acceptor is needed to create lipophilic environment in order to diffuse through the aqueous hydrophilic apoplast and reach the surface polymerisation site. Due to their apoplastic localisation and to their ability to bind lipids in their hydrophobic cavity, LTPs have been often suggested for this function (Sterk et al., 1991; Pyee et al., 1994; Suh et al., 2005). However, no experimental data has confirmed so far the transport function of plant LTPs. Concerning polymerisation of the cutin precursors, two mechanisms can be actually considered, i.e. a chemical or an enzymatic transesterification process. Chemical trans-esterification could be catalysed by the slightly acidic pH of the apoplastic compartment, but is, in a first attempt, a mechanism too slow to respond at the rapid growth of cuticle during plant development.

Actually, two enzymes are good candidates to perform the polymerization of cutin precursors. First of all, BODYGUARD, an epidermis-specific and extracellular α/β hydrolase-protein whose interruption in Arabidopsis induces deformation of epidermal cell walls and an irregular deposition of cutin. Furthermore, the mutant accumulates more cutin and wax monomers than the wild plants (Kurdyukov et al., 2006). BDG could either play a role of cutin syntethase or participate to the control of cell proliferation and differentiation of the epidermis (Kurdyukov et al., 2006). In this later hypothesis, the modification of the epidermis cells would limit the formation of the cuticle layer (Kurdyukov et al., 2006; Pollard et al., 2008).

The second candidates are proteins of the GDSL family of lipase-hydrolases. These enzymes are located in the cell walls close to the cuticular membranes (Reina et al., 2007) and their expression is under the control of WIN1, the transcription factor that regulates cutin deposition (Kannangara et al., 2007).

However, except a role in tolerance to biotic and abiotic stress (Oh et al., 2005; Naranjo et al., 2006; Hong et al., 2008) no experiment has been conducted to relate these proteins with building of cuticular barriers. Although a transferase activity was demonstrated for microbial protein homologue so that this family is also called the GDSL108 motif lipase-acylhydrolase family (Akoh et al., 2004), only their hydrolytic activities towards lipid ester analogues were tested (Oh et al., 2005; Hong et al., 2008).

### SUMMARY OF THE INVENTION

This invention is based on the isolation and characterization of GDSL1 the main GDSL-motif lipase-acylhydrolase protein expressed in the exocarp of developing tomato fruit (*Solanum lycopersicum var*). Silencing of this protein leads to major alteration in the formation of cutin and an increase of the permeability of fruit cuticle.

This invention is also based on the characterization of GDSL3 expressed in the seeds of developing tomato fruit (*Solanum lycopersicum var*) and GDSL2 expressed in all tomato fruit tissues but only at early developmental stages.

Finally, this invention is also based on sequence alignments between plant species to identify the subfamily of GDSL-motif lipase-acylhydrolase proteins involved in fruit development, particularly in fruit cuticle polymerization.

Plants of the invention with reduced cuticle around the fruits have the advantage that they allow more cost-effective production of juice, puree and coulis of fruits.

Plants of the invention with more brilliant cuticle around the fruits have also the advantage to be more attractive for consumers.

Thus, a first object of the invention is an isolated plant GDSL-lipase polypeptide differentially expressed between the different steps of the plant development, preferably of the fruit development, and/or the different tissues of the plant, preferably of the fruit, comprising the sequences SEQ ID NO:1, SEQ NO:2, SEQ ID NO:3, SEQ ID NO:4 and SEQ ID NO:5 in this order from the N-terminal to the C-terminal ends

A second object of the invention is an isolated polynucleotide comprising a nucleic acid molecule encoding for a polypeptide of the invention, analogs, paralogs or orthologs thereof.

A third object of the invention is a vector comprising a polynucleotide of the invention.

A fourth object of the invention is a host cell genetically engineered with the polynucleotide of the invention, or with the vector of the invention.

A fifth object of the invention is a monoclonal antibody directed to a polypeptide of the invention.

A sixth object of the invention is a plant or part of plant genetically engineered to not express at least partially an isolated plant GDSL-lipase polypeptide of the invention. A seventh object of the invention is a tomato plant or a part of a tomato plant genetically engineered to not express at least partially an isolated plant GDSL-lipase polypeptide selected from the group consisting of GDSL1 (SEQ ID NO:6), GDSL2 (SEQ ID NO:7) or GDSL3 (SEQ ID NO:8).

An eighth object of the invention is a tomato fruit genetically engineered to not express at least partially GDSL1 (SEQ ID NO:6).

A ninth object of the invention is a method for obtaining a plant mutant carrying at least a point mutation which suppresses at least partially the expression of an isolated plant GDSL-lipase polypeptide of the invention, said method comprising the following steps:
a. A mutagenesis treatment of seeds with a chemical mutagen such as Ethyl methanesulfonate (EMS);
b. Self-fertilization of the resulting M1 plants;
c. Extraction of DNA samples of the M2 individuals for mutational screening;
d. The DNA samples are pooled and arrayed in microtiter plates, and the pools are amplified using primers specific of a polypeptide of the invention;
e. Amplification products are incubated with the CEL I or ENDO I endonuclease;
f. Cleavage products are electrophoresed to identify punctual mutations in said polypeptide.

A tenth object of the invention is the use of a part of plant which is a fruit genetically engineered to not express at least partially an isolated plant GDSL-lipase polypeptide of the invention or a part of a plant mutant which is a fruit carrying at least a point mutation which suppresses at least partially the expression of an isolated plant GDSL-lipase polypeptide of the invention obtainable by the TILLING method of the invention for preparing puree, juice or coulis of said fruit.

An eleventh object of the invention is the use of a part of plant genetically engineered to not express at least partially an isolated plant GDSL-lipase polypeptide of the invention or a part of a plant mutant carrying at least a point mutation which suppresses at least partially the expression of an isolated plant GDSL-lipase polypeptide of the invention obtainable by the TILLING method of the invention for preparing an extract.

An twelfth object of the invention is the use of a tomato fruit genetically engineered to not express at least partially an isolated plant GDSL-lipase polypeptide of the invention or a tomato fruit carrying at least a point mutation which suppresses at least partially the expression of an isolated plant GDSL-lipase polypeptide of the invention obtainable by the TILLING method of the invention for preparing canned peeled tomatoes, tomato juice, tomato paste and tomato ketchup.

### DETAILED DESCRIPTION OF THE INVENTION

### Polypeptides

The first object of the invention relates to an isolated plant GDSL-lipase polypeptide differentially expressed between the different steps of the plant development, preferably of the fruit development and/or the different tissues of the plant, preferably of the fruit, comprising the sequences SEQ ID NO:1, SEQ NO:2, SEQ ID NO:3, SEQ ID NO:4 and SEQ ID NO:5 in this order from the N-terminal to the C-terminal ends, and its orthologs, and derivatives thereof.

The terms "polypeptide", "peptide" or "protein" are used interchangeable throughout this specification.

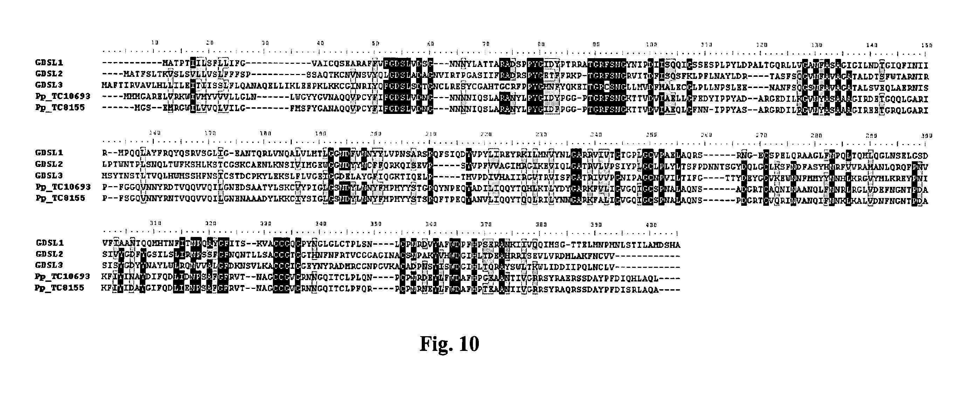

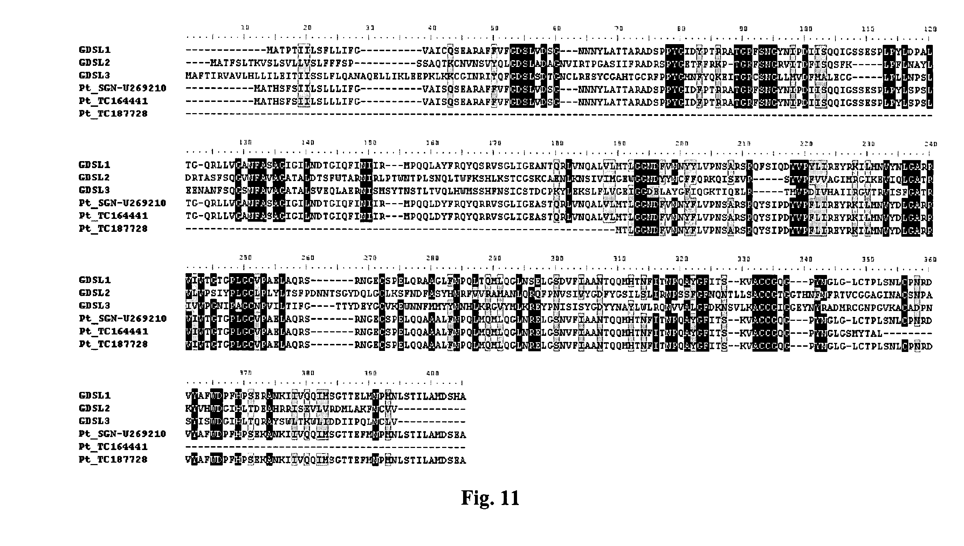

The isolated plant GDSL-lipase polypeptides according to the invention thus comprise five conserved blocks defined as SEQ ID NO:1 to 5.This is illustrated by the sequence alignments of figures 10 and 11. The isolated plant GDSL-lipase polypeptides according to the invention have also a common function which is defined as a differential expression between the different steps of the plant development, preferably of the fruit development and/or the different tissues of the plant, preferably of the fruit.

Preferably, SEQ ID NO:1 is SEQ ID NO:9, more preferably is SEQ ID NO:29. Preferably, SEQ ID NO:2 is SEQ ID NO:10, more preferably is SEQ ID NO: 14. Preferably, SEQ ID NO:3 is SEQ ID NO:11, more preferably is SEQ ID NO:15, even more preferably SEQ ID NO:17. In one embodiment, SEQ ID NO:3 is SEQ ID NO:30.

Preferably, SEQ ID NO:4 is SEQ ID NO:12, more preferably is SEQ ID NO:16. Preferably, SEQ ID NO:5 is SEQ ID NO:13.

Preferably, the isolated plant GDSL-lipase polypeptide of the invention comprises the sequences SEQ ID NO:9, SEQ NO:10, SEQ ID NO:11, SEQ ID NO:12 and SEQ ID NO:13 in this order from the N-terminal to the C-terminal ends.

Preferably, the isolated plant GDSL-lipase polypeptide of the invention comprises the sequences SEQ ID NO:32, SEQ NO:14, SEQ ID NO:33, SEQ ID NO:16 and SEQ ID NO:13 in this order from the N-terminal to the C-terminal ends.

Preferably, the isolated plant GDSL-lipase polypeptide of the invention comprises a Y amino acid in the sequence separating SEQ ID NO:1 and SEQ ID NO:2, more preferably a PYG triad (SEQ ID NO:18), even more preferably SEQ ID NO:23.. Preferably, the isolated plant GDSL-lipase polypeptide of the invention comprises SEQ ID NO:19 in the sequence separating SEQ ID NO:2 and SEQ ID NO:3, more preferably SEQ ID NO:20 or SEQ ID NO:21.

Preferably, the isolated plant GDSL-lipase polypeptide of the invention comprises a CC duplicate in the sequence separating SEQ ID NO:4 and SEQ ID NO:5, more preferably SEQ ID NO:22.

Preferably, the isolated plant GDSL-lipase polypeptide of the invention comprises the sequences SEQ ID NO:1, SEQ ID NO:18, SEQ NO:2, SEQ ID NO:19, SEQ ID NO:3, SEQ ID NO:4, SEQ ID NO:22 and SEQ ID NO:5 in this order from the N-terminal to the C-terminal ends.

More preferably, the isolated plant GDSL-lipase polypeptide of the invention comprises the sequences SEQ ID NO:9, SEQ ID NO:23, SEQ NO:10, SEQ ID NO:20 or 21, SEQ ID NO:11, SEQ ID NO:12, SEQ ID NO:22 and SEQ ID NO:13 in this order from the N-terminal to the C-terminal ends.

In one embodiment, an isolated plant GDSL-lipase polypeptide according to the invention comprises the sequences SEQ ID NO:1, SEQ NO:2, SEQ ID NO:3, SEQ ID NO:4 and SEQ ID NO:5 in this order from the N-terminal to the C-terminal ends separated by sequences which present at least 60%, preferably at least 70%, and more preferably at least 80%, the most preferably at least 90% with the sequence SEQ ID NO:6, SEQ ID NO:7 or SEQ ID NO:8 its orthologs, and derivatives thereof. To determine the percent homology of two amino acid sequences (e.g., one of the sequences encoded by a nucleic acid of the invention and a mutant form thereof) or of two nucleic acids, the sequences are aligned for optimal comparison purposes (e.g., gaps can be introduced in the sequence of one protein or nucleic acid for optimal alignment with the other protein or nucleic acid). The amino acid residues or nucleotides at corresponding amino acid positions or nucleotide positions are then compared. When a position in one sequence is occupied by the same amino acid residue or nucleotide as the corresponding position in the other sequence, then the molecules are homologous at that position (i.e., as used herein amino acid or nucleic acid "homology" is equivalent to amino acid or nucleic acid "identity"). The percent homology between the two sequences is a function of the number of identical positions shared by the sequences (i.e., % homology = numbers of identical positions/total numbers of positions x 100).

Preferably, an isolated plant GDSL-lipase polypeptide according to the invention comprises:
(a) a polypeptide sequence selected in the group consisting of SEQ ID NO:6, SEQ ID NO:7 or SEQ ID NO:8, or
(b) a polypeptide sequence which is at least 60%, preferably at least 70%, and more preferably at least 80%, the most preferably at least 90% identical to the polypeptide of (a), said polypeptide being differentially expressed between the different steps of the plant development, preferably of the fruit development and/or the different tissues of the plant, preferably of the fruit, or
(c) a polypeptide sequence being a fragment of any one of (a) to (b), wherein said fragment or biologically active portion thereof is differentially expressed between the different steps of the plant development, preferably of the fruit development and/or the different tissues of the plant, preferably of the fruit.

More preferably, an isolated plant GDSL-lipase polypeptide according to the invention comprises:
(a) the polypeptide sequence SEQ ID NO:6, or
(b) a polypeptide sequence which is at least 60%, preferably at least 70%, and more preferably at least 80%, the most preferably at least 90% identical to the polypeptide of (a), said polypeptide being capable of altering cutin polymerization, or
(c) a polypeptide sequence which is at least 60%, preferably at least 70%, and more preferably at least 80%, the most preferably at least 90% identical to the polypeptide of (a), said polypeptide having an esterase activity, or
(d) a polypeptide sequence being a fragment of any one of (a) to (c), wherein said fragment or biologically active portion thereof is capable of altering cutin polymerization, or
(e) a polypeptide sequence being a fragment of any one of (a) to (c), wherein said fragment or biologically active portion thereof has an esterase activity.

A polypeptide according to the invention may be produced by culturing a cell transformed with a nucleotide sequence of this invention (in the sense orientation), allowing the cell to synthesize the protein and then isolating the protein, either as a free protein or as a fusion protein, depending on the cloning protocol used, from either the culture medium or from cell extracts. Alternatively, the protein can be produced in a cell-free system.
Preferably, an isolated plant GDSL-lipase polypeptide is GDSL1 of SEQ ID NO:6
Preferably, an isolated plant GDSL-lipase polypeptide is GDSL2 of SEQ ID NO:7 Preferably, an isolated plant GDSL-lipase polypeptide is GDSL3 of SEQ ID NO:8 Preferably, a polypeptide according to the invention has a lipase, hydrolase and/or esterase activity.
More preferably, an isolated plant GDSL-lipase polypeptide according to the invention has an esterase activity.

**Table B summarizes SEQ ID NO:1 to 22 with X is any one of amino acids except when X is specified below**

| | | |
|---|---|---|
| **BLOC 1** | **SEQ ID NO: 1** | **XGDSLXDXG** |
| | **(the first X is F or L)** | **F** |
| | | **L** |
| | **SEQ ID NO:9** | **FGDSLVDSG** |
| | **SEQ ID NO:29** | **RAFFVFGDSLVDSGNN** |
| | **SEQ ID NO: 18** | **PYG** |
| | **SEQ ID NO:23** | **RADXPPYG** |
| **BLOC 2** | **SEQ ID NO:2** | **TGRXXXGXXXXD** |
| | | **FSN** |
| | | **CCD** |
| | | **Y** |
| | **SEQ ID NO:10** | **TGRXSNGXXXXD** |
| | | **F I** |
| | | **C H** |
| | **SEQ ID NO: 14** | **TGRFSNGXNIXD** |
| | **SEQ ID NO:19** | **GXNXAXAXXXXL** |
| | | **F S G** |
| | | **Y V A** |
| | **SEQ ID NO:20** | **GXNFASAGIGIL** |
| | **SEQ ID NO:21** | **GXNFAVAGATAL** |
| **BLOC 3** | **SEQ ID NO:3** | **XXXX** |
| | | **GGND** |
| | | **SDE** |
| | **SEQ ID NO:11** | **GGNDXXN** |
| | **SEQ ID NO:30** | **GGNDFVNN** |
| | **SEQ ID NO:15** | **LVLXXLGGNDXXN** |
| | **SEQ ID NO:17** | **LVNXALVLXTXGGNDFVNNY** |
| **BLOC 4** | **SEQ ID NO:4** | **XGAXRXXVXXXXPXGCXP** |
| | | **V** |
| | | **I** |
| | **SEQ ID NO:12** | **LGAXRVXVXXXXPLGCXP** |
| | **SEQ ID NO:16** | **LGARXVXVTGTGPLGC** |
| | **SEQ ID NO:22** | **ACCGXG** |
| **BLOC 5** | **SEQ ID NO:5** | **CXXXXXXXXWDXXHXX** |
| | | **N PS** |
| | | **D LT** |
| | **SEQ ID NO:13** | **LCPNRXXYAFWDXFHPXEXA** |

**Table A shows the positions of SEQ ID NO:1 to 5 in GDSL1, GDSL2 and GDSL3.**

| | **Position of SEQ ID NO:** | | | | |
|---|---|---|---|---|---|
| **Protein** | **1** | **2** | **3** | **4** | **5** |
| **GDSL1** | **29-37** | **63-74** | **163-166** | **206-223** | **313-328** |
| **GDSL2** | **38-46** | **74-85** | **173-176** | **211-228** | **331-346** |
| **GDSL3** | **52-60** | **89-100** | **188-191** | **226-243** | **342-357** |

### Expression

In the context of the present application, "differentially expressed" means an expression different in terms of cDNA level, mRNA level or protein level.

The different steps of the plant development comprise germination, stem elongation, flowering, fruit and seed or grain maturation, ear emergence in cereals.

The different steps of the fruit development are expressed in "days post anthesis" (DPA). The early step fruit development is approximately between 1 and 12 DPA. The maturation step fruit development is approximately between 13 and 25 DPA. The end of the fruit development is approximately between 35 and 45 DPA.

The different tissues of the plant comprise vegetative tissues and reproductive organs, in particular fruit, inflorescence, anther, petal, sepal, young leaf, mature leaf, stem, hair and root.

The different fruit tissues comprise seed, locular tissue, columella, mesocarp and exocarp.

Preferably, an isolated plant GDSL-lipase polypeptide according to the invention is differentially expressed during the fruit development. More preferably, an isolated plant GDSL-lipase polypeptide according to the invention is expressed during early fruit development or during fruit ripening.

Preferably, an isolated plant GDSL-lipase polypeptide according to the invention is specifically expressed in one or two fruit tissue(s). More preferably, an isolated plant GDSL-lipase polypeptide according to the invention is specifically expressed in the exocarp of the fruit or in the grains.

Preferably, an isolated plant GDSL-lipase polypeptide according to the invention is excreted outside the cell where it is expressed.

### Species

Preferably, an isolated plant GDSL-lipase polypeptide according to the invention is isolated from a commercially important crop including Asteraceae (including the food crops lettuce, chicory, globe artichoke, sunflower, yacon, safflower), Cucurbitaceae (commonly known as gourds or cucurbits and includes crops like cucumbers, squashes (including pumpkins), luffas, melons and watermelons), Brassica (including swedes, turnips, kohlrabi, cabbages, brussels sprouts, cauliflower, broccoli, mustard seed and oilseed rape), Leguminous Crops (including dry beans, dry broad beans, dry peas, chickpea, garbanzo, bengal gram dry cowpea, black-eyed pea, blackeye bean, pigeon pea, toor, cajan pea, congo bean, lentil, bambara groundnut, earth pea vetch, common vetch Lupins, soybean, peanut) Chenopodiaceae (spinach). Liliaceae (unions, leek), Apiaceae (carrots), Grain crops (rice, barley, wheat, oats, corn), Solanaceae (including tomato, courgette, aubergine, hot pepper, sweet pepper, eggplant), fruit trees (apple tree, orange tree, Clementine tree), pine tree or vineyard.

More preferably, an isolated plant GDSL-lipase polypeptide according to the invention is isolated from a fruit-tree, from a cereal plant, for example cotton plant, rice plant, corn plant, oats plant, wheat plant, from vegetable plants, for example lettuce plant, courgette plant, aubergine plant, tomato plant, hot pepper plant or sweet pepper plant and from pine tree, vineyard.

### Polynucleotides

Another object of the invention is an isolated polynucleotide comprising a nucleic acid molecule encoding for a polypeptide of the invention.

Preferably, an insolated polynucleotide of the invention comprises SEQ ID NO:24 encoding SEQ ID NO:6, analogs, paralogs or orthologs thereof.

Preferably, an insolated polynucleotide of the invention comprises SEQ ID NO:25 encoding SEQ ID NO:7, analogs, paralogs or orthologs thereof.

Preferably, an insolated polynucleotide of the invention comprises SEQ ID NO:26 encoding SEQ ID NO:8, analogs, paralogs or orthologs thereof.

As used herein, the term "nucleic acid molecule" is intended to include DNA molecules (e.g., cDNA or genomic DNA) and RNA molecules (e.g., mRNA) and analogs of the DNA or RNA generated using nucleotide analogs. This term also encompasses untranslated sequence located at both the 3' and 5' ends of the coding region of a gene: at least about 1000 nucleotides of sequence upstream from the 5' end of the coding region and at least about 200 nucleotides of sequence downstream from the 3' end of the coding region of the gene. The nucleic acid molecule can be single-stranded or double-stranded, but preferably is double-stranded DNA. An "isolated" nucleic acid molecule is one, which is substantially separated from other nucleic acid molecules, which are present in the natural source of the nucleic acid. Preferably, an "isolated" nucleic acid is substantially free of sequences that naturally flank the nucleic acid (i.e., sequences located at the 5' and 3' ends of the nucleic acid) in the genomic DNA of the organism, from which the nucleic acid is derived.

As used herein, the term "orthologs" refers to two nucleic acids from different species, but that have evolved from a common ancestral gene by speciation. Normally, orthologs encode proteins having the same or similar functions.

The term "polynucleotide" as used in accordance with the present invention relates to a polynucleotide comprising a nucleic acid molecule which encodes a polypeptide having a biologically activity as specified above.

Preferably, the polynucleotide of the invention comprises a sequence selected from the group consisting of SEQ ID NO:24, SEQ ID NO:25 or SEQ ID NO:26. Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, also comprise a nucleic acid sequence characterized in that the sequence can be derived from the aforementioned specific nucleic acid sequences shown in SEQ ID NO:24, SEQ ID NO:25 or SEQ ID NO:26 by at least one nucleotide substitution, addition and/or deletion whereby the variant nucleic acid sequence shall still encode a polypeptide having a biological activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 x sodium chloride/sodium citrate (= SSC) at approximately 45°C, followed by one or more wash steps in 0.2 x SSC, 0.1 % SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 x SSC (pH 7.2). If organic solvent is present in the abovementioned buffer, for example 50% formamide, the temperature under standard conditions is approximately 42°C. The hybridization conditions for DNA:DNA hybrids are, preferably, 0.1 x SSC and 20°C to 45°C, preferably between 30°C and 45°C. The hybridization conditions for DNA: RNA hybrids are, preferably, 0.1 x SSC and 30<0>C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991 , "Essential Molecular Biology: A Practical Approach", IRL Press at Oxford University Press, Oxford. Alternatively, polynucleotide variants are obtainable by PCR-based techniques such as mixed oligonucleotide primer-based amplification of DNA, i.e. using degenerated primers against conserved domains of the polypeptides of the present invention.

Further, variants include polynucleotides comprising nucleic acid sequences which are at least 70%, at least 75%, at least 80%, at least 85%, at least 90%, at least 95%, at least 98% or at least 99% identical to the nucleic acid sequences shown in SEQ ID NO:24, SEQ ID NO:25 or SEQ ID NO:26. A polynucleotide comprising a fragment of any of the aforementioned nucleic acid sequences is also encompassed as a polynucleotide of the present invention. The fragment shall encode a polypeptide which still has a biological activity as specified above. A fragment as meant herein, preferably, comprises at least 20, at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences. Variant polynucleotides as referred to in accordance with the present invention may be obtained by various natural as well as artificial sources. For example, polynucleotides may be obtained by in vitro and in vivo mutagenesis approaches using the above mentioned mentioned specific polynucleotides as a basis. Moreover, polynucleotide being homologs or orthologs may be obtained from various plant species.

The present invention, thus, also encompasses an oligonucleotide which specifically binds to the polynucleotides of the present invention. Binding as meant in this context refers to hybridization by Watson-Crick base pairing discussed elsewhere in the specification in detail. An oligonucleotide as used herein has a length of at most 100, at most 50, at most 40, at most 30 or at most 20 nucleotides in length which are complementary to the nucleic acid sequence of the polynucleotides of the present invention. The sequence of the oligonucleotide is, preferably, selected so that a perfect match by Watson-Crick base pairing will be obtained. The oligonucleotides of the present invention may be suitable as primers for PCR-based amplification techniques. Moreover, the oligonucleotides may be used for RNA interference (RNAi) approaches in order to modulate and, preferably down- regulate, the activity of the polypeptides encoded by the polynucleotides of the present invention. As used herein, the term "RNA interference (RNAi)" refers to selective intracellular degradation of RNA used to silence expression of a selected target gene, i.e. the polynucleotide of the present invention. RNAi is a process of sequence-specific, post- transcriptional gene silencing in organisms initiated by double-stranded RNA (dsRNA) that is homologous in sequence to the gene to be silenced. The RNAi technique involves small interfering RNAs (siRNAs) that are complementary to target RNAs (encoding a gene of interest) and specifically destroy the known mRNA, thereby diminishing or abolishing gene expression. RNAi is generally used to silence expression of a gene of interest by targeting mRNA, however, any type of RNA is encompassed by the RNAi methods of the invention. Briefly, the process of RNAi in the cell is initiated by long double stranded RNAs (dsRNAs) being cleaved by a ribonuclease, thus producing siRNA duplexes. The siRNA binds to another intracellular enzyme complex which is thereby activated to target whatever mRNA molecules are homologous (or complementary) to the siRNA sequence. The function of the complex is to target the homologous mRNA molecule through base pairing interactions between one of the siRNA strands and the target mRNA. The mRNA is then cleaved approximately 12 nucleotides from the 3' terminus of the siRNA and degraded. In this manner, specific mRNAs can be targeted and degraded, thereby resulting in a loss of protein expression from the targeted mRNA. A complementary nucleotide sequence as used herein refers to the region on the RNA strand that is complementary to an RNA transcript of a portion of the target gene. The term "dsRNA" refers to RNA having a duplex structure comprising two complementary and anti-parallel nucleic acid strands. Not all nucleotides of a dsRNA necessarily exhibit complete Watson-Crick base pairs; the two RNA strands may be substantially complementary. The RNA strands forming the dsRNA may have the same or a different number of nucleotides, with the maximum number of base pairs being the number of nucleotides in the shortest strand of the dsRNA. Preferably, the dsRNA is no more than 49, more preferably less than 25, and most preferably between 19 and 23, nucleotides in length. dsRNAs of this length are particularly efficient in inhibiting the expression of the target gene using RNAi techniques. dsRNAs are subsequently degraded by a ribonuclease enzyme into short interfering RNAs (siRNAs). RNAi is mediated by small interfering RNAs (siRNAs). The term "small interfering RNA" or "siRNA" refers to a nucleic acid molecule which is a double stranded RNA agent that is complementary to i.e., able to base-pair with, a portion of a target RNA (generally mRNA), i.e. the polynucleotide of the present invention being RNA. siRNA acts to specifically guide enzymes in the host cell to cleave the target RNA. By virtue of the specificity of the siRNA sequence and its homology to the RNA target, siRNA is able to cause cleavage of the target RNA strand, thereby inactivating the target RNA molecule. Preferably, the siRNA which is sufficient to mediate RNAi comprises a nucleic acid sequence comprising an inverted repeat fragment of the target gene and the coding region of the gene of interest (or portion thereof). Also preferably, a nucleic acid sequence encoding a siRNA comprising a sequence sufficiently complementary to a target gene is operatively linked to a expression control sequence. Thus, the mediation of RNAi to inhibit expression of the target gene can be modulated by said expression control sequence. Preferred expression control sequences are those which can be regulated by a exogenous stimulus, such as the tet operator whose activity can be regulated by tetracycline or heat inducible promoters. Alternatively, an expression control sequence may be used which allows tissue- specific or expression of the siRNA or expression at defined timepoints in development. The complementary regions of the siRNA allow sufficient hybridization of the siRNA to the target RNA and thus mediate RNAi. In mammalian cells, siRNAs are approximately 21-25 nucleotides in length (see Tuschl et al. 1999 and Elbashir et al. 2001 ). The siRNA sequence needs to be of sufficient length to bring the siRNA and target RNA together through complementary base- pairing interactions. The siRNA used with a seed specific expression system e.g. under control of the USP promoter of the invention may be of varying lengths. The length of the siRNA is preferably greater than or equal to ten nucleotides and of sufficient length to stably interact with the target RNA; specifically 15-30 nucleotides; more specifically any integer between 15 and 30 nucleotides, most preferably 15, 16, 17, 18, 19, 20, 21 , 22, 23, 24, 25, 26, 27, 28, 29, and 30. By "sufficient length" is meant an oligonucleotide of greater than or equal to 15 nucleotides that is of a length great enough to provide the intended function under the expected condition. By "stably interact" is meant interaction of the small interfering RNA with target nucleic acid (e.g., by forming hydrogen bonds with complementary nucleotides in the target under physiological conditions). Generally, such complementarity is 100% between the siRNA and the RNA target, but can be less if desired, preferably 91 %, 92%, 93%, 94%, 95%, 96%, 97%, 98%, or 99%. For example, 19 bases out of 21 bases may be base-paired. In some instances, where selection between various allelic variants is desired, 100% complementary to the target gene is required in order to effectively discern the target sequence from the other allelic sequence. When selecting between allelic targets, choice of length is also an important factor because it is the other factor involved in the percent complementary and the ability to differentiate between allelic differences. Preferably, the oligonucleotides used for RNAi approaches are selected from the 5<'> or 3' untranslated region of a cDNA corresponding to a polynucleotide of the present invention.

Also in a preferred embodiment, the oligonucleotide of the present invention can be used for the generation of or as a micro RNA (miRNA). These miRNAs are single-stranded RNA molecules of preferably 20 to 25, more preferably 21 to 23 nucleotides in length capable of regulating gene expression. miRNAs are physiologically encoded by genes that are transcribed from DNA but not translated into protein (non-coding RNA). Rather, they are processed from primary transcripts known as pri-miRNA to short stem-loop structures called pre-miRNA and finally to functional miRNA. The mature miRNA molecules are partially complementary to one or more messenger RNA (mRNA) molecules. Due to this complementary regions, they are capable of binding to a given target mRNA and to subsequently down- regulate expression thereof. Preferably, miRNAs to be used in accordance with the present invention comprise sequence stretches complementary to the polynucleotide sequences of the present invention referred to above. More preferably, the sequence stretches are between 20 and 30 nucleotides, more preferably, between 20 and 25 nucleotides and, most preferably, between 21 and 23 nucleotides in length. The miRNAs, preferably, are capable of binding to complementary sequences of the coding regions or of the 3<'>or the 5<'>UTR sequences of the polynucleotides of the invention.

More preferably, the oligonucleotide of the present invention, thus, comprises a sequence of at least 15 nucleotides in length complementary to the nucleic acid sequence of the polynucleotide of the invention. Most preferably, the said oligonucleotide is capable of down regulating the expression of the said polynucleotide, preferably either by functioning as a double stranded RNAi molecule or as a single stranded miRNA molecule. Downregulation as meant herein relates to a statistically significant reduction of the mRNA detectable in a cell, tissue or organism or even to a failure to produce mRNA in detectable amounts at all. This also includes the reduction of of the stability of mRNA encoding all or a part of any sequence described herein. Moreover, downregulation also encompasses an impaired, i.e. significantly reduced, production of protein from RNA sequences encoding all or a part of any sequence or even the absence of detectable protein production.

The present invention provides antisense oligonucleotides and polynucleotides complementary to an isolated plant GDSL-lipase polypeptide according to the invention or one of its orthologs from another plant which hybridizes with it under high stringency conditions. Such antisense oligonucleotides should be at least about six nucleotides in length to provide minimal specificity of hybridization and may be complementary to one strand of DNA or mRNA encoding an isolated plant GDSL- lipase polypeptide according to the invention or a portion thereof, or to flanking sequences in genomic DNA which are involved in regulating the expression of said isolated plant GDSL-lipase polypeptide according to the invention. The antisense oligonucleotide may be as large as 100 nucleotides and may extend in length up to and beyond the full coding sequence for which it is antisense. The antisense oligonucleotides can be DNA or RNA or chimeric mixtures or derivatives or modified versions thereof, single stranded or double stranded. The action of the antisense oligonucleotide may result in alteration, primarily inhibition, of the expression of said isolated plant GDSL-lipase polypeptide according to the invention in cells.

SiRNAs used by the inventors to inhibit the expression of GDSL1 (SEQ ID NO:6) have the sequences SEQ ID NO:49 and SED ID NO:50 and are also an object of the present invention.

### Vector

A vector according to the invention comprises a polynucleotide according to the invention.

Vectors can be plasmids preferably, or may be viral or other vectors known in the art to replicate and express genes encoded thereon in plant cells or bacterial cells. The vector becomes chromosomally integrated such that it can be transcribed to produce the desired antisense senescence-induced lipase RNA. Such plasmid or viral vectors can be constructed by recombinant DNA technology methods that are standard in the art. For example, the vector may be a plasmid vector containing a replication system functional in a prokaryotic host. Alternatively, the vector may be a plasmid containing a replication system functional in *Agrobacterium.*

In order for a newly inserted gene or DNA sequence to be expressed, resulting in production of the protein which it encodes, or in the case of antisense DNA, to be transcribed, resulting in an antisense RNA molecule, the proper regulatory elements should be present in proper location and orientation with respect to the gene or DNA sequence. The regulatory regions may include a promoter, a 5'-non-translated reader sequence and a 3'-polyadenylation sequence as well as enhancers and other regulatory elements.

Promoter regulatory elements that are useful in the vector according to the invention in combination with a nucleic acid according to the invention include any plant promoter in general.

The vector of the present invention, preferably, further comprises selectable markers for propagation and/or selection in a host. The vector may be incorporated into a host cell by various techniques well known in the art. If introduced into a host cell, the vector may reside in the cytoplasm or may be incorporated into the genome. In the latter case, it is to be understood that the vector may further comprise nucleic acid sequences which allow for homologous recombination or heterologous insertion. Vectors can be introduced into prokaryotic or eukaryotic cells via conventional transformation or transfection techniques.

### Host cells

A host cell according to the invention is genetically engineered with a polynucleotide of the invention or with a vector of the invention.

Host cells are primary cells or cell lines derived from multicellular organisms such as plants or animals. Furthermore, host cells encompass prokaryotic or eukaryotic single cell organisms (also referred to as microorganisms), e.g. bacteria or fungi including yeast or bacteria. Primary cells or cell lines to be used as host cells in accordance with the present invention may be derived from the multicellular organisms, preferably from plants.

Also provided in accordance with the present invention is a method for the manufacture of a polypeptide having the biological activity of a polypeptide encoded by a polynucleotide of the present invention comprising:
(a) expressing the polynucleotide of the present invention in a host cell; and
(b) obtaining the polypeptide encoded by said polynucleotide from the host cell. The polypeptide may be obtained, for example, by all conventional purification techniques including affinity chromatography, size exclusion chromatography, high pressure liquid chromatography (HPLC) and precipitation techniques including antibody precipitation. It is to be understood that the method may - although preferred -not necessarily yield an essentially pure preparation of the polypeptide. It is to be understood that depending on the host cell which is used for the aforementioned method, the polypeptides produced thereby may become post-translationally modified or processed otherwise.

### Antibodies

The invention also relates to a monoclonal antibody directed to a polypeptide according to the invention.

Antibodies against the polypeptides of the invention can be prepared by well known methods using a purified polypeptide according to the invention or a suitable fragment derived therefrom as an antigen. A fragment which is suitable as an antigen may be identified by antigenicity determining algorithms well known in the art. Such fragments may be obtained either from the polypeptide of the invention by proteolytic digestion or may be a synthetic peptide. Preferably, the antibody of the present invention is a monoclonal antibody, a polyclonal antibody, a single chain antibody, a human or humanized antibody or primatized, chimerized or fragment thereof. Also comprised as an antibody by the present invention is a bispecific antibody, a synthetic antibody, an antibody fragment, such as Fab, Fv or scFv fragments etc., or a chemically modified derivative of any of these. The antibody of the present invention shall specifically bind to the polypeptide of the invention, i.e. it shall not significantly cross react with other polypeptides or peptides. Specific binding can be tested by various well known techniques. Antibodies or fragments thereof can be obtained by using methods which comprise the fusion of mouse myeloma cells to spleen cells derived from immunized mammals. The antibodies can be used, for example, for the immunoprecipitation, immunolocalization or purification (e.g., by affinity chromatography) of the polypeptides of the invention as well as for the monitoring of the presence of said variant polypeptides, for example, in recombinant organisms, and for the identification of compounds interacting with the proteins according to the invention.

### Plant

The invention also relates to a plant or part of plant which does not express at least partially an isolated plant GDSL-lipase polypeptide according to the invention. Preferably, the plant or part of plant according to the invention is genetically engineered to not express at least partially an isolated plant GDSL-lipase polypeptide according to the invention.

The quantity of expressed GDSL-lipase polypeptide according to the invention can be decreased by a decrease in cDNA or RNA levels corresponding to the GDSL-lipase polypeptide according to the invention or by a decrease in protein level whereas the cDNA and RNA levels did not change.

In one embodiment, the GDSL-lipase polypeptide according to the invention level is decreased by RNAi.

The quantity of expressed GDSL-lipase polypeptide according to the invention can also be decreased by a decrease in cDNA or RNA or protein level of a precursor of said GDSL-lipase polypeptide according to the invention.

In one embodiment, the level of a precursor of a GDSL-lipase polypeptide according to the invention level is decreased by RNAi.

The invention also relates to a plant or part of plant which expresses an isolated plant GDSL-lipase polypeptide according to the invention having an abnormal activity. By "a modified isolated plant GDSL-lipase polypeptide according to the invention to not have a normal activity" is meant:
- an isolated plant GDSL-lipase polypeptide according to the invention the differential expression of which is altered, and/or
- an isolated plant GDSL-lipase polypeptide according to the invention the role in cutin polymerization of which is altered, and/or
- an isolated plant GDSL-lipase polypeptide according to the invention the esterase, lipase or hydrolase activity of which is altered.

In one embodiment, the polypeptide is genetically modified to have an abnormal activity. For example, a punctual mutation or a deletion or an insertion of nucleotides has been introduced in the part of the corresponding gene responsible of the activity and this modification implies an abnormal activity.

In another embodiment, another polypeptide involved in the activity pathway of the polypeptide of the invention is modified and this modification inhibits the activity of the polypeptide of the invention.

As used herein, the term "plant" refers to either a whole plant, a plant part, a plant cell or a group of plant cells.

Preferably, a plant according to the invention is a transgenic plant.

A transgenic plant is defined herein as a plant which is genetically modified in some way. The altered genetic material may encode a protein comprising a regulatory or control sequence or may be or include an antisense sequence or encode an antisense RNA or siRNA which inhibits the DNA or mRNA sequence or portion thereof responsible of the expression of the polypeptide of which a modification of the expression is foreseen.

A "transgene" or "transgenic sequence" is defined as a foreign gene or partial sequence which has been incorporated into a transgenic plant.

Transgenic plants made in accordance with the present invention may be prepared by DNA transformation using any method of plant transformation known in the art. Plant transformation methods include direct cocultivation of plants, tissues or cells with *Agrobacterium tumefaciens* or direct infection; direct gene transfer into protoplasts or protoplast uptake; electroporation; particle bombardment; injection into meristematic tissues of seedlings and plants; injection into protoplasts of cultured cells and tissues. Generally a complete plant is obtained from the transformation process. Plants are regenerated from protoplasts, callus, tissue parts or explants, etc.

Expression of an isolated plant GDSL-lipase polypeptide according to the invention can be assayed in a conventional immunoassay method using a specific antibody as described herein.

Preferably, a plant according to the invention is a tomato plant or a part of a tomato plant genetically engineered to not express at least partially an isolated plant GDSL-lipase polypeptide selected from the group consisting of GDSL1 (SEQ ID NO:6), GDSL2 (SEQ ID NO:7) or GDSL3 (SEQ ID NO:8).

More preferably, a plant according to the invention is a tomato fruit genetically engineered to not express at least partially GDSL1 (SEQ ID NO:6).

Another object of the present invention relates to a method for producing a transgenic plant, preferably a tomato plant, with reduced cuticle around the fruits. Transgenic plants may be obtained in various manners including e.g. silencing of the polynucleotide according to the invention by introduction of antisense, sense suppression or RNAi (RNA interference) nucleic acid constructs into the plants, or by knock-out of the polynucleotide according to the invention by homologous recombination. In another preferred embodiment, the transgenic plants with non-dehiscent anthers may be obtained by knock-out of the polynucleotide according to the invention by homologous recombination. Homologous recombination allows introduction in a genome of a selected nucleic acid at a defined selected position. Methods for performing homologous recombination in plants have been described not only for model plants (Offringa et al, 1990 EMBO J. 9(10):3077-84) but also for crop plants, for example rice (Iida and Terada, Curr Opin Biotechnol. 2004 April; 15(2): 132- 8).

### TILLING

Another object of the present invention is a TILLING (Targeted Induced Local Lesions IN Genomes) method for obtaining a plant mutant carrying at least a point mutation which suppresses at least partially the expression of an isolated plant GDSL-lipase polypeptide according to the invention, said method comprising the following steps:
a. A mutagenesis treatment of seeds with a chemical mutagen such as Ethyl methanesulfonate (EMS);
b. Self-fertilization of the resulting M1 plants;
c. Extraction of DNA samples of the M2 individuals for mutational screening;
d. The DNA samples are pooled and arrayed in microtiter plates, and the pools are amplified using primers specific of a polypeptide as claimed in claims 1 to 6;
e. Amplification products are incubated with the CEL I or ENDO1 endonuclease;
f. Cleavage products are electrophoresed to identify punctual mutations in said polypeptide.

Methods for TILLING are well known in the art (McCallum et al reviewed by Stemple). The TILLING mutagenesis technology is useful to generate and/or identify, and to eventually isolate mutagenised variants of a polynucleotide according to the invention. TILLING also allows selection of plants carrying such mutant variants. TILLING combines high-density mutagenesis with high-throughput screening methods. The steps typically followed in TILLING are: (a) a mutagenesis treatment of seeds with a chemical mutagen such as Ethyl methanesulfonate (EMS) or N-ethyl-N-nitrosourea (ENU); (b) backcrossing the plants for at least one generation; (c) preparation of DNA from tissue samples of the plants and (d) pooling of DNAs from individuals; (e) PCR amplification of a region of interest, (f) detection of mutant PCR products (g) identification of the mutant individual and (h) optionally, sequencing of the mutant PCR. Initially in the method detection of mutant PCR products in step (f) and optionally (g) was performed by denaturation and annealing to allow formation of heteroduplexes and subsequent detection of heteroduplexes by DHPLC (Denaturing High Perfomance Liquid Chromatography). The method was made higher throughput by using the restriction enzyme CEL-I or ENDO-I combined with a gel based system to identify mutations (Colbert et al., 2001, Plant Physiol. 126(2):480-4). Other methods for detection or identification of single base mutations in the target gene that may be used in methods of TILLING include e.g. resequencing DNA as has been described (see e.g. Slade et al., 2005, Nat Biotechnol. 23(1):75-81) or massive parallel sequencing (e.g. of Roche / 454 Life Sciences performed on a GS 20 Genome Sequencer) to produce sequence information for a given locus in thousands of individuals, optionally combined with a 3D pooling strategy (see e.g the Keypoint(TM) technology at www.keygene.com). Mutagenesis may be performed by radiation or with a chemical mutagen such as EMS (Lightner and Caspar, 1998, "Seed mutagenesis of Arabidopsis", In: Methods in Molecular Biology: Arabidopsis Protocols (eds. J. M. Martinez-Zapater and J. Salinas), pp. 91-103. Totowa, New Jersey: Humana Press) or ENU (Draper et al., 2004, Methods Cell Biol. 2004;77:91-112).

Preferably, the mutation in the GDSL1-allele cause the allele to be a truncation or missense allele.

### USES

Another object of the invention is the use of a part of plant which is a fruit genetically engineered to not express at least partially an isolated plant GDSL-lipase polypeptide of the invention or a part of a plant mutant which is a fruit carrying at least a point mutation which suppresses at least partially the expression of an isolated plant GDSL-lipase polypeptide of the invention obtainable by the TILLING method of the invention for preparing puree, juice or coulis of said fruit.

Preferably, said fruit is a tomato.

Another object of the invention is the use of a part of plant genetically engineered to not express at least partially an isolated plant GDSL-lipase polypeptide of the invention or a part of a plant mutant carrying at least a point mutation which suppresses at least partially the expression of an isolated plant GDSL-lipase polypeptide of the invention obtainable by the TILLING method of the invention for preparing an extract, preferably without using organic solvents.

Preferably, said use is the use of a part of plant according to the invention which is a wheat or barley grain for preparing malt.

Another object of the invention is the use of a tomato fruit genetically engineered to not express at least partially an isolated plant GDSL-lipase polypeptide of the invention or a tomato fruit carrying at least a point mutation which suppresses at least partially the expression of an isolated plant GDSL-lipase polypeptide of the invention obtainable by the TILLING method of the invention for preparing canned peeled tomatoes, tomato juice, tomato paste or tomato ketchup.

The different possible uses of the plants and part of plants of the invention are linked to their following advantages:
- their visual aspect is modified so that they show a shiny appearance appealing to the consumer
- their epiderm thickness is modified so that their sensorial quality as a fresh product is more attractive to the consumer - e.g. reducing the amount of peel in tomato fruit so that children are not rebutted by the feeling of thick fruit peel in their mouth when eating cherry tomato fruit
- their epiderm thickness and composition are modified in order to facilitate the extraction of compounds of interest - e.g. reducing the cuticle thickness and composition on wheat or barley grains in order to facilitate the use of malt during the brewing process - e.g. reducing the cuticle thickness and composition so that the use of organic solvents is not necessary anymore
- their epiderm thickness and composition are modified in order to reduce the amount of wastes (peel, cuticle) - e.g. reducing the amount of peel wastes generated when processing tomato to obtain canned peeled tomatoes, tomato juice, tomato paste and tomato ketchup
- their epiderm thickness and composition are modified in order so that surface composition of the cuticle is modified and does not allow pathogens to attack the plant - e.g. suppression of host recognition by the pathogen in order to suppress host-pathogen interactions detrimental to the host plant

Having now generally described the invention, the same will be more readily understood through reference to the following figures and examples which are provided by way of illustration, and are not intended to be limiting to the present invention, unless specified.

### FIGURES

Figure 1: Sequence alignment of the GDSL1, GDLS2 and GDSL3 proteins.
Figure 2: Expression profiles of 3 genes coding for putative GDSL lipase in tomato plant organs. Real time RT-PCR experiments were performed to assess transcript abundance of GDSL1 (A), GDSL2 (B) and GDSL3 (C). IF, immature fruit; Sta,
stamen; Pet, petals; YL, young leaves; ML, mature leaves; Ste, stem; Po, pollen; R,
roots. AU = arbitrary units. Vertical bars represent standard deviation (n=3).
Figure 3: Temporal expression of GDSL lipase coding genes along fruit development.
Figure 4: Expression pattern of GDSL lipase coding genes in fruit tissues.
Figure e 5: Exocarp cuticule thickness in fruit of WT and P35SSlGDSL1 RNAi plants. (A) Cross section of exocarp on fruits from WT and transgenic lines 10, 4, 3 and 17 (light microscopy). Coloration with Sudan Red revealed cutine polymers and cutinised cells of the first exocarp cells layer encased in cutin. Arrows 1 and 2 represent the positions of thickness measurements. These measurements were performed (B) between two epidermic cells (widest part of the cuticle) or (C) above one epidermic cell (smallest part of the cuticle). Vertical bars represent standard deviation (n=60), * and *** indicate a significant difference of thickness with WT samples (t test, respectively P<0.05 and P<0.001).

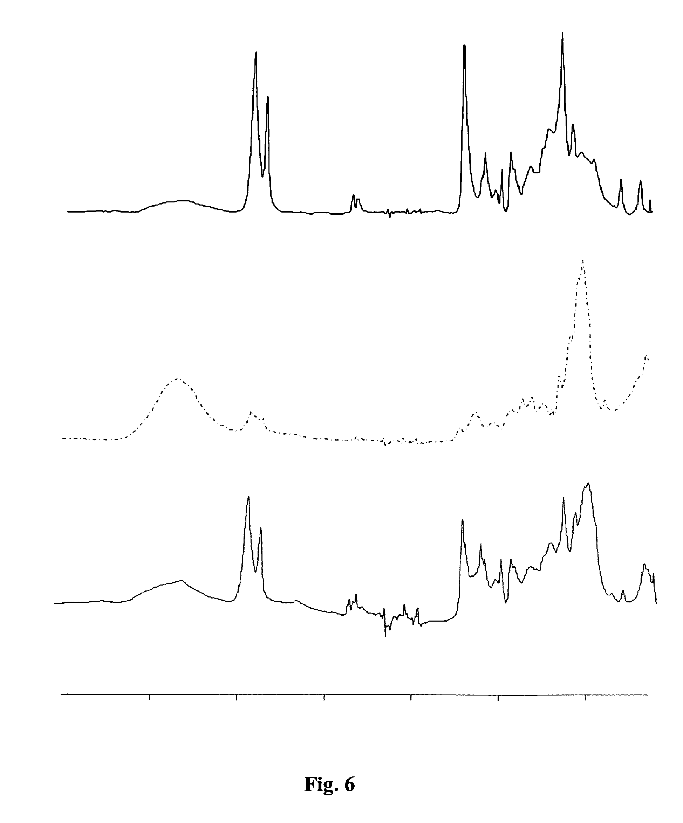

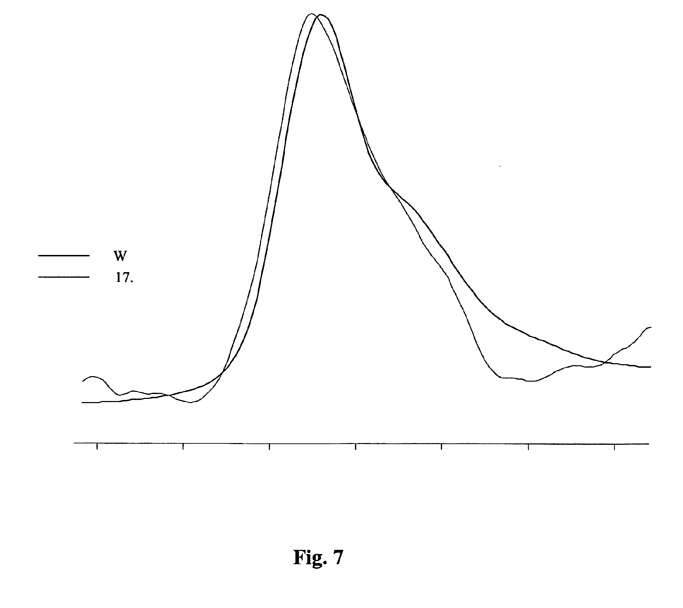

Figures 6 and 7 : ATR FTIR

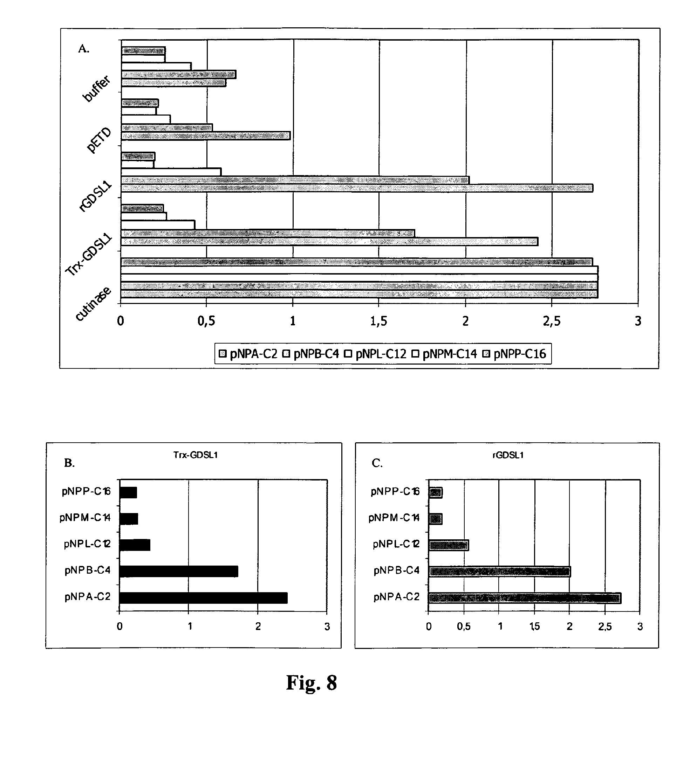

Figure 8: Hydrolytic activity of Thr-GDSL1 fusion protein (B.) and rGDSL1 recombinant protein (C.). Purified Thr-GDSL1 fusion protein and cleaved rGDSL1 protein were assayed for their hydrolytic activity on various para-nitrophenyl (pNP) derivates, pNP acetate (pNPA, C₂), pNP butyrate (pNPB, C₄), pNP laurate (pNPL, C₁₂), pNP myristolate (pNPM, C₁₄), and pNP palmitate (pNPP, C₁₆). Fungal cutinase was used as positive control and protein solubilisation buffer and protein extraction from pETD without insert were used as negative controls (A.). Measurements were performed after 120 min incubation at 37°C by optical density reading at 405 nm with spectrophotometer.

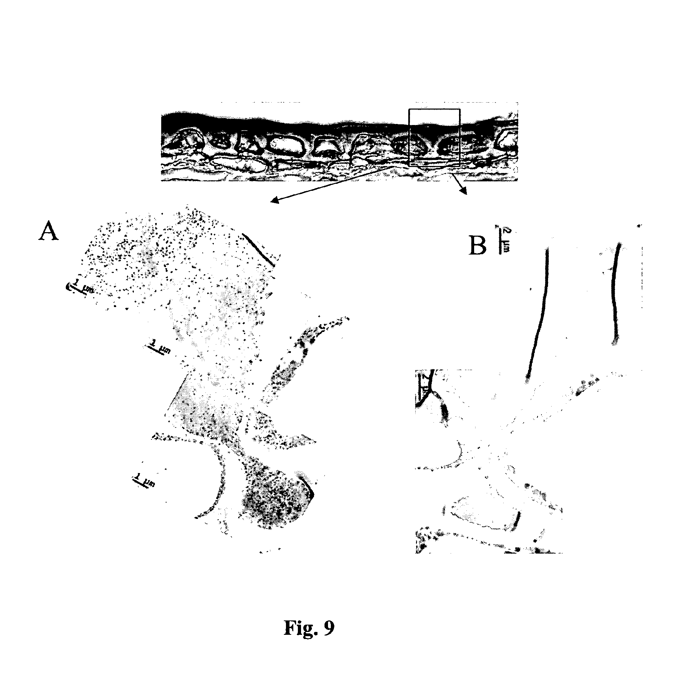

Figure 9 : immunolocalization of GDSL in Wt tomato exocarp (A). Blank control without GDSL1 antibody
Figure 10 : Alignement GDSL1, 2 et 3 de Tomate avec 2 séquences de piment
Figure 11 :Alignement GDSL1, 2 et 3 de Tomate avec 3 séquences de Pomme de terre

### EXAMPLES

### Example: Isolation and characterization of GDSL1

### 1. Material and methods

### 1.1. Tomato fruit cuticle isolation

Cuticles were isolated from commercial red ripe tomato fruits of *Solanum lycopersicum.* Isolation of cuticles was carried out using a modified protocol (Petracek and Bukovac, 1995). Briefly, peels were removed from tomato red fruits and incubated for two weeks at room temperature in an enzymatic solution of fungal cellulase (2% v/v, Sigma, St Louis, Missouri, USA) and fungal pectinase (2% v/v, Sigma) containing 1 mM NaN₃ to prevent microbial growth. The enzymatic solution was changed four times during the 2 weeks. Cuticular matrices were finally separated from the epidermis cell walls by extensive washing in distilled water and dried on nylon tissue. Cuticles were scanned and their surface was assessed using the Image J software (http://rsb.info.nih.gov/ij/).

### 1.2. Cloning, expression, purification of recombinant GDSL1

### 1.2.1. Cloning of GDSL1

The gdsl1 gene was overexpressed in Escherichia coli using pET expression system. Specific primers were designed (GDSL3': 5'TAAAGCTTATGCATGTGAAT3'= SEQ ID NO:27 and GDSL5': 5'ATCCATGGGGCAAAGTGAAGCTAGGGCATTT3' = SEQ ID NO:28) and used to amplify and clone the coding region of gdsl1 with the NcoI and HindIII restriction sites (underlined sequences). cDNA was subcloned into expression vector pETD. The recombinant plasmid, pETD-GDSL1, was introduced into E. coli BL21 competent cells. E. coli transformed with pETD vector without insert was used as negative control in all study. Clones with plasmid were selected using 100 µg/ml ampicillin on Luria Bertani (LB) medium. A second construction was also used to overexpress and purify GDSL1 in E Coli using the same pET expression system. Specific primer were designed (GDSL6 5' ATCCATGGGGCAAAGTGAAGCTAGGGCATTT = SEQ ID n°29) GDSL7 5'ATCTCGAGTGCATGTGAATCCATAGCCAG = SEQ ID n°30) and used to amplify and clone the coding region of gdsl1 with the NcoI and XhoI restriction sites. Cloning and production of the protein was conducted in the same conditions as described above.

### 1.2.2. Production and purification of recombinant protein in Escherichia coli

For production, cultures were grown in LB medium added by 100 µg/ml ampicillin at 37°C to early exponential phase (optical density at 600 nm [OD600] of 0.6), and then IPTG109 (isopropyl-β-D-thiogalactopyranoside) was added to a final concentration of 0.5 mM.

Subsequent protein expression was allowed to proceed for 3 h at 37°C. Bacterial cells were harvested by centrifugation at 14000 rpm for 20 min at 4°C. Dried pellets were stored at -20°C until using.

Thioredoxin-GDSL1 (Thx-GDSL1) fusion protein was extracted from culture pellets in solubilized buffer (Na-Phosphate 20 mM, NaCl 500 mM and urea 8 M). After purification by metal chelate affinity chromatography with Ni-NTA His resin (Invitrogen), Thr-GDSL1 was submitted to chemical cleavage and recombinant GDSL1 (rGDSL1) protein then purified passing another time on nickel affinity chromatography.

For the second construction, the protein was extracted from culture pellets with a Tris NaCl (50 mM , 200mM, pH7,5) buffer. Protein purification was conducted through a Ni-NTA resin.

### 1.3. Hydrolase activities

### 1.3.1. Assay of lipase activity of GDSL1 recombinant in E.Coli

To monitor esterase activity of GDSL1 in E. coli, a modified tributyrin agar diffusion assay (Rashid et al., 2001) was used. Briefly, LB agar medium containing tributyrin (1% v/v), IPTG (50 µM) and ampicillin (50 µg/mL) was emulsified by sonication after autoclaving and poured into Petri dishes. In this agar plates, several wells (5 mm diameter) were aseptically punched. Ten microliters of fresh culture were loaded in the wells and examined for their ability to form clear halos, indicating esterase activity. A culture of E. coli containing pETD without GDSL1 was used as negative control.

### 1.3.2. Lipase/esterase colorimetric enzyme assay

Lipase and esterase activity was assessed by measuring the absorbance of the pnitrophenol (pNP) (Ruiz et al., 2004). Different substrates were chosen as a function of their chain length: p-nitrophenyl acetate (pNPA, C2), p-nitrophenylbutyrate (pNPB, C4), pnitrophenyl laurate (pNPL, C12), p-nitrophenyl myristate (pNPM, C14) and p-nitrophenyl palmitate (pNPP, C16). Each substrate was dissolved at 20 mM in isopropanol and sonicated for 3 min. Each solution was diluted in phosphate-Triton X100 buffer in a ratio 1:10 (v/v) under gentle agitation to obtain a final concentration of 2 mM pNP, 10% isopropanol, 1,2% Triton X100 and 50 mM Na-phosphate buffer pH 7.8. 1 mL of this solution was mixed with 1 mL of enzyme solubilized in 20 mM Na-phophate pH 7.8 and 150 mM NaCl. 1 mL of pET32b in the same buffer and 1 mL of this buffer alone were used as negative control and 25 µL of cutinase from Fusarium graminearum and 975 µL of buffer as positive control. The absorbance of pNP was recorded at 405 nm for each samples during 30 min with measurement every 5 min. (see Figure 8)

### 1.4. Plant material and growth conditions

Cherry tomato plants, *Solanum lycopersicum var. cerasiformae* West Virginia 106', were grown under standard green house conditions. Medium-sized fruit tomato plants, *Solanum lycopersicum var. Ailsa craig,* were grown in a growth chamber with a light period of 14 h in light and 10 h in dark under a thermoperiod of 25°C/20°C and watered with a nutrient solution three times a day. For in vitro culture, seeds were decontaminated in a calcium hypochlorite (4% p/v) and Tween 20 (0.1% v/v) solution for 20 min, rinsed in distilled water, dried at room temperature and sown in a Murashige and Skoog . (MS, 1.1 g/L) medium with agar (8 g/L) containing (transgenic plants) or not (wild type) kanamycin (300 µg/L).

### 1.5. Plant transformation

cDNA fragment was obtained by reverse transcription (SuperScript II Reverse transcriptase, Invitrogen, life technology) on 2 µg of total RNA from tomato fruit. Two fragments of 509 bp and 148 bp, located respectively in the 5' region and in the 3'-untranslated region of GDSL1, were amplified by PCR using ExTaq DNA polymerase and gene specific primers (table I). Each purified DNA fragment was introduced in the Gateway system entry vector (pDONR 201) and then transferred as an inverted repeat under control of 35S promoter in the RNAi destination vector (pK7GWIWG2), which confers kanamycin resistance to transformed plants (Karimi et al., 2002). After verification by sequencing, constructions were used to transform cherry tomato plants ('West Virginia 106') via *Agrobacterium tumefaciens* strain GV3101 (Hamza and Chupeau, 1993). Primary transfomant were used to analyze expression profile and homozygous plants were used to study phenotypic modifications.

**Table I**

| **primer** | **5'-3' sequence** |
|---|---|
| GDSL1s1 | AAAAAGCAGGCTCCAAAATGGCCACACCTACT |
| GDSL1s2 | AAAAAGCAGGCTTTTTTGTTGCTAATTTTTGCCTA |
| GDSL1as3 | AGAAAGCTGGGTTGACAAAAATCATTGCCTCCA |
| GDSL1as4 | AGAAAGCTGGGTCAACAATATCCACACTCCACCCTA |
| *att*B1 | GGGGACAAGTTTGTACAAAAAAGCAGGCTNN |
| *att*B2 | GGGGACCACTTTGTACAAGAAAGCTGGGTNN |

### 1.6. Gene expression

### 1.6.1. Sequence analysis

GDSL sequences are indexed in the Institute of Genomic Research (TIGR) Plant Transcript Assemblies Database (http://plantta.jcvi.org/search.shtml, Solanum lycopersicum, release 5, 01/06/2007) and in the SOL Genomics Network (http://www.sgn.cornell.edu/) under accession numbers as follows: GDSL1 (TA37529_4081 ; SGN-U566206), GDSL2 (TA38323_4081 ; SGN-U585129) and GDSL3 (A41795_4081 ; SGN-U318275). Sequence comparisons were carried out with ClustalW software (http://www.ebi.ac.uk/Tools/clustalw2 /index.html).

### 1.6.2. Tissue collection and RNA extraction

Expression analysis of the three GDSL sequences was carried out on wild type *Ailsa craig* plants in order to characterize various gene expressions: (i) in different tissues:
fruit at 20 days after anthesis (DPA), inflorescence, anther, petal, sepal, young leaf, mature leaf, stem, hair, and root, (ii) during fruit development: 4, 8, 12, 20, 27, 35, 40 and 45 DPA, (iii) in separated fruit tissues: seed, locular tissue, columella, mesocarp and exocarp, at different stages of fruit development: 12, 20, 27 and 35 DPA. Another analysis compared GDSL expression profiles in cherry tomato between silencing and wild-type plants in leaves, flowers and fruits. For each tissue analyzed, the material collected was immediately frozen in liquid nitrogen and stored at -80°C. Total tomato RNA was isolated from leaf tissue using standard trizol RNA extraction protocol (Life Technologies, Basel, Switzerland) and further DNase treated (Turbo DNA-free Kit, Ambion/Applied Biosystems, Courtaboeuf, France). Four extractions were performed on each collected sample. RNA was separated by electrophoresis on a 1.2% agarose gel to check for degradation.

### 1.6.3. Quantification of gene expression using qRT-PCR

One µg of DNase-treated total RNA was reverse transcribed as described by Lemaire-Chamley et al. (Lemaire-Chamley et al., 2005). The RT-product was diluted 10-fold in water, and aliquots of 2 µL were used in real time PCR reaction (25 iL final volume) in the presence of 0.2 µM clone-specific primers using the iQTM SYBR Green Supermix (Bio-Rad, Marne La Coquette, France) according to the manufacturer's instruction. Real-time PCR was performed on a iCyclerTM iQ (Bio-Rad) with the following parameters: a first step of denaturation at 95°C for 3 min, followed by a 40 amplification cycles with denaturation step at 95°C for 30 s, and an hybridation/synthesis step at 60°C for 30 s. During last step, temperature increased of 60 to 97°C with 0.5°C amplification all 10 s in order to built fusion curve. Data acquisition and analysis was done using the iCyclerTM iQ software (version 3.0a, Biorad). EiF4α (BT013166), b-tubulin (DQ205342) and actin were used as housekeeping genes allowing calculation of relative expression of interest genes: *gdsll , gdls2, gdsl3* using Genes (Gene Expression Analysis fro iCycler IQ Real Time PCR detection system) Excel applet from Bio-Rad. All specific primers used in quantitative RT-PCR are recapitulated in table II.

**Table II : Specific primer sequences used for quantitative real time PCR**

| **gene** | **forward primer** | **reverse primer** |
|---|---|---|
| *gdsl1* | GCAACACTTCATGTTGTGCTA CTT = SEQ ID NO:37 | CAATATCACACTCCACCCTAC AAA = SEQ ID NO:38 |
| *gdsl2* | GCCAAATTCAACTGCGTCGTT T = SEQ ID NO:39 | TCTCCTTCGCTACAACGTGGA A= SEQ ID NO:40 |
| *gdsl3* | GGGTGGCCAATTACAAAATG= SEQ ID NO:41 | ACTCACACACATCACATAAA GATGC= SEQ ID NO:42 |
| β*-tubulin* | AACCTCTCGTGGATCACAGC= SEQ ID NO:43 | GGCAGAAGCTGTCAGGTAAC G= SEQ ID NO:44 |
| *EiF4α* | AGTGGACGATTTGGAAGGAA G= SEQ ID NO:45 | GCTCCTCGATTACGACGTTG= SEQ ID NO:46 |
| *actin* | GGACTCTGGTGATGGTGTTAG = SEQ ID NO:47 | CCGTTCAGCAGTAGTGGTG= SEQ ID NO:48 |

### 1.7. Subcellular localization of GDSL1

Thin ultramicrotome sections (100 nm) of Wt tomato exocarp were incubated in 3% (w/v) BSA in 10mM PBS (pH 7.2) and incubated for 1h with the polyclonal antibody diluted in 10mM phosphate buffer saline supplemented with 1% BSA and 0,05% tween 20. After extensive washing, the section were incubated for 1h with a gold-conjugated antibody ( Aurion, NL). Labelling was intensified though the use of silver enhancement kit (Aurion, NL) according to the manufacturer instruction. After washing, the grids were stained with 2% uranyl acetate. Electrographs were taken with a Jeol 100S TEM electron microscope. (see Figure 9)

### 1.8. Effect ofRNAi on plant

### 1.8.1. Analysis of cutin monomers

Cuticular waxes were twice extracted from isolated cuticles of tomato in 10 mL of methylene chloride. The resulting solution of cuticular waxes was spiked with 10 µg of tetracosane (Sigma) as internal standard. Extracted cutins were transesterified by treatment with 15% boron trifluoride in methanol (Sigma) for 16 h at 70°C including 5 µg of heptadecanoic acid as internal standard. The organic products was extracted with CH₂Cl₂ (3x5 mL), dried over anhydrous sodium sulfate and dried under an N₂ stream. Prior to GC analysis, wax and cutin samples were sylilated with 50 µL of bis-(trimethylsilyl) trifluoroacetamide containing 1% trimethylchlorosilane (Sigma) at 70°C for 60 min. The qualitative composition was studied with capillary GC-MS (Thermo trace GC ultra) equipped with (DB5ms, 30m x0.32 mm, 0.1 mm [J&W]) and a mass spectrometric detector (Thermo DSQII, 70eV, m/z 50-700). For cutin analyses, GC was carried out in an oven temperature programmed for 1 min at 90°C, 40 °C.min-1 up to 200°C, 1 min at 200°C, 3°C.min-1 up to 320°C. For waxes analyses, GC was carried out in an oven temperature programmed for 2 min at 50°C, 40 °C.min-1 up to 220°C, 2 min at 200°C, 3°C.min-1 up to 320°C, 30 min at 320°C. The quantitative composition was studied using GC-FID (Hewlett Packard 5890) under the same GC conditions, but H2 was used as carrier gas at 2 mL.min⁻¹.

### 1.8.2. FTIR analysis.

FTIR was performed on a Nicolet Magna 550 ThermoElectron spectrometer (Madison, WI) equipped with a KBr beam splitter and a liquid-nitrogen-cooled mercurycadmium-telluride detector. Each spectrum is the result of the average of 200 scans in the 4000 to 600 cm⁻¹ range at 2 cm⁻¹ resolution apodized with a Happ-Genzel function. For ATR infrared spectroscopy, pieces (at least 0.5 x 0.5 cm) of cuticle and cutin (external and internal sides) were steadily spread on the surface of ZnSe or germanium crystals.

### 1.8.3. Toluidine blue staining

A toluidine blue assay was performed to identify defects in the permeability of fruit cuticles in wild type and transformed tomato plants (Tanaka et al., 2004; Hovav et al., 2007). Fruits were placed in cup filled with an aqueous solution of toluidine blue at 1% (Sigma, St Louis, MO, USA). Approximately a quarter of the fruit surface was immersed for 4 h in the toluidine blue solution. Afterwards fruits were washed with distilled water to remove the excess of dye.

### 1.8.4. Light microscopy and cuticle thickness of transformed plants

Slices of 20 DPA fruits cuticle from wt, 3.1.9, 10.10, 4.2, 17.3 plants were realized using a cryostat (Icrom HM500OM, Microm France). Samples were frozen and rolled up on it-self in cryoprotector. Cross-sections with a thickness of 20 µm were then obtained and observed with a light microscope (Leica DMRB, Leica) and a magnification of X20.

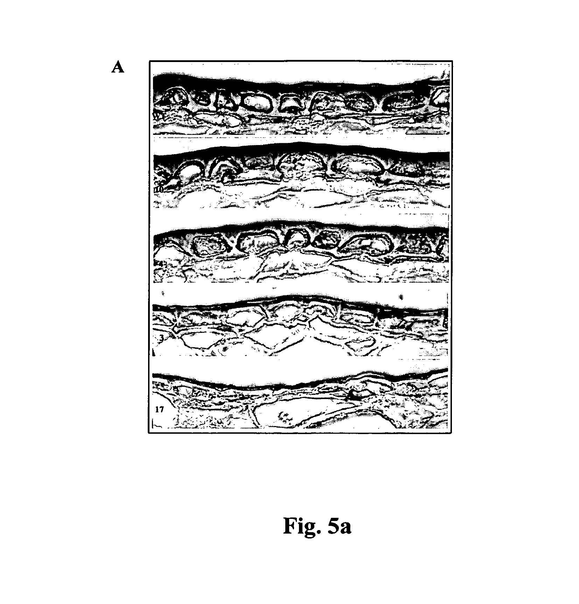

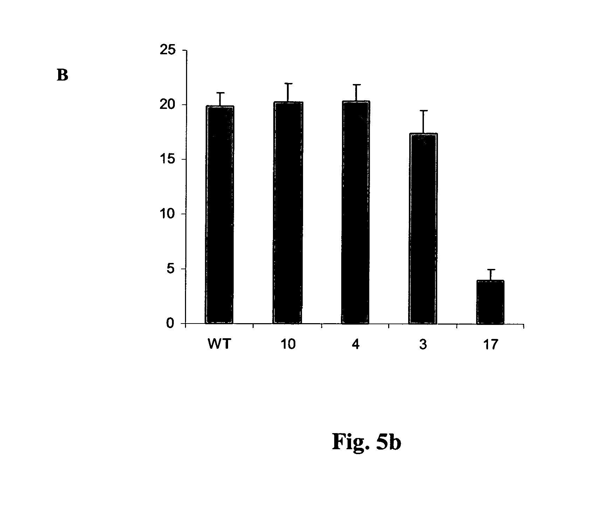

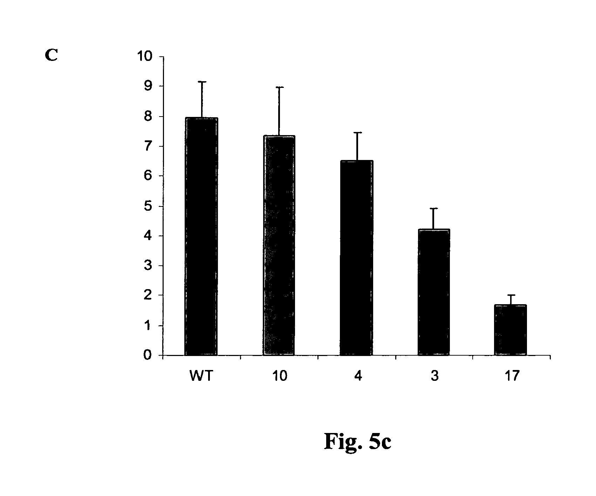

Cuticle thickness was measured from 3 different fruits (10 measures per fruit). For some transformant and wt plants (20 DPA and breaker +7 days), fruit exocarpe (including cuticule) were cut from two different fruits and immersed in 0.1 M phosphate buffer (pH 7.2) containing 3% paraformaldehyde and 0.1% glutaraldehyde for 16 h at 4°C. The samples were rinsed, dehydrated in ethanol series and embedded in LR white hard grade (Agar Aids). Ultramicrotom (Microm RMC MT 700) sections (1 µm) were obtained and were observed on a light microscope (Leica DMRB, Leica). Sections were examined on a LEICA DMRD. A band-pass filter 450-490 nm was used as excitation filters and primary fluorescence was detected at > 515 nm. (see Figure 5)

### 1.8.5. Atomic force microscopy

Cutin samples were fixed on a metallic support using double-side sticky tape. AFM height, phase and error-signal images were acquired in air using an Autoprobe CP Park Scientific Instrument (Sunnyvale, CA). AFM images were recorded in the Tapping mode using conventional pyramidal silicon nitride cantilevers obtained from Digital Instrument (Santa Barbara, CA). All the Tapping mode images were acquired at the lowest possible stable scanning force, less than 10 nN. Different surface areas were scanned from each sample 80 µm x 80 µm, 40 µm x 40 µm and 2 µm x 2 µm). The mean surface roughness (RMS) parameter was deduced from the height images recorded with a surface of 2 µm x 2 µm

### 1.8.6. Scanning electron microscopy

The morphology of air dried cuticles extracted from the wild type and 17.3 plants were prepared as previously described (Bargel and Neinhuis, 2005) and were characterized by scanning electron microscopy (SEM). The samples were sputter coated with a fine gold layer (approx 100-200 A) before recording the SEM images using a Leo-1450VP scanning electron microscope (LEO Electron Microscopy Ltd, Cambridge, UK) and low-voltage conditions.

### 2. Results

### 2.1. Isolation, and sequence analysis of GDSL1 cDNA clone

Preliminary proteomic study of tomato cutin allowed us to isolate an abundant GDSL-motif lipase-acylhydrolase called GDSL1 (TA37529_4081, http://plantta.jcvi.org/search.shtml) from tomato cutin. The presence of this protein was confirmed by SDS-PAGE and immunoblotting of SDS extracts of cuticle powder using the specific antibodies produced against the corresponding recombinant protein. The cuticular localization of GDSL-motif lipase-acylhydrolase is in agreement with previous immunocytochemical studies on *Agave Americana* (Reina et al., 2007).

GDSL1 cDNA clone was then isolated from tomato exocarp by RT-PCR using specific primers derived from the nucleotidic sequence available on SGN tomato database (SGN-U313619, http://www.sgn.cornell.edu/). The full length cDNA (SEQ ID NO: 24, 1042 bp) contains an open reading frame coding a 362 amino acid protein (SEQ ID NO:27). GDSL1 precursor shows a theoretical pI of 7.55 and a theorical molecular mass of 39694.26 daltons. Peptide signal is predicted by SignalP software and cleavage site is situated between Cys19 and Gln20. For mature GDSL1 protein, theoretical pI and molecular mass are respectively 7.75 and 37688 Da. Extracellular localisation of GDSL1 was predicted for GDSL1 by TargetP 1.1 software (http://www.cbs.dtu.dk/services/targetP). Extracellular localization seems to be a common feature of GDSL. Indeed, 107 putative gdsl coding genes have been recently listed (Ling, 2008) in *Arabidopsis thaliana.* Among the corresponding proteins, 99 were predicted as extracellular localized.

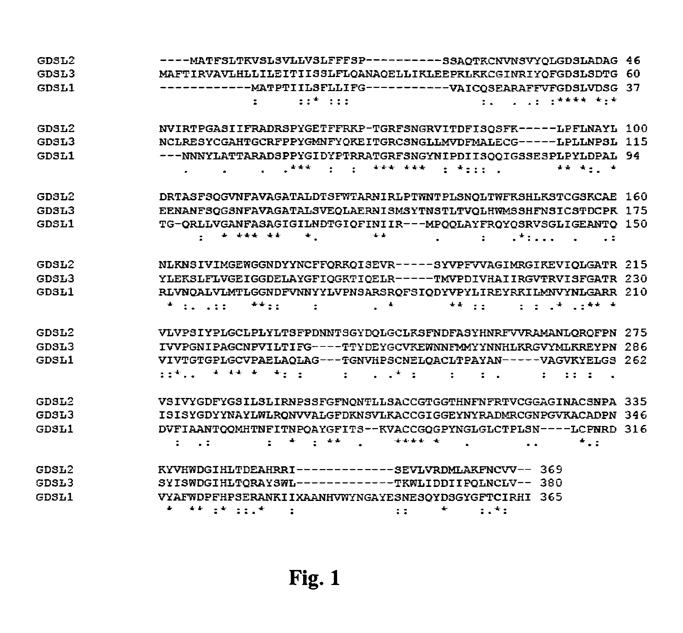

GDSL-motif lipase-acylhydrolase proteins belong to a large multigenic family, characterized by a GDSL motif, five conserved blocks sequences (figures 1,10 and 11). Three amino-acids are described as typical catalytic triad for esterase and lipase activity (Ling et al., 2006): serine (S), aspartic acid (D) and histidine (H). In order to characterize GDSL lipase genes expressed during early tomato fruit development, we searched for proteins annotated as GDSL-motif lipase-acylhydrolase and/or presenting sequence homologies to GDSL1 in tomato SGN database. This screen allowed identifying 38 tomato unigenes.

Further selection was based on the presence of the full GDSL motif (20 remaining unigenes) and on the expression of these genes during early fruit developing (7 remaining unigenes). We focused our attention on three unigenes (SGN-U313619: GDSL1, SGNU315324:GDSL2, and SGN-U318275: GDSL3) since they were represented by more than one EST in early tomato fruit databases. Comparison of amino acid sequences (figure 1) reveals that GDSL1 shows 23 and 19.9% of identity with GDSL2 and GDSL3, respectively.

Alignment of amino acid sequences (figure 1) show conservation of GDSL lipase typical motif (glycine, aspartic acid, serine and leucine) between GDSLs. The SDH triad is also observed among tomato GDSLs. Serine of GDSL motif is present in bloc I, histidine residue in block V. As described in the literature, the aspartic acid residue is either localized in block III (Upton and Buckley, 1995) for GDSL1 and GDSL2 or in block V just upstream histidine (Akoh et al., 2004) for GDSL1, GDSL2 and GDSL3.

GDSL1 sequence was also compared to other lipases and hydrolases with GDSL motif of different plants presented in literature. GDSL1 shows moderate percentage of identity in amino acids with AgaSGNH of *Agave americana* (67.5%, AAS75127) (Reina et al., 2007) and LTL1 of *Arabidopsis thaliana* (64.9%, AAG51758) (Naranjo et al., 2006). Identity percentage is lower for other aligned plant proteins, CaGLIP1 of *Capsicum annuum* (33%. AAX20033) (Hong et al., 2008), CaGL1 of *C*. *annuum* (32.7%, AA223955) (Kim et al., 2008), GLIP1 of *Arabidopsis thaliana* (29.8%, NP_198915) (Oh et al., 2005), ENOD8 of *Medicago sativa* (26.1%, AAB41547) (Pringle and Dickstein, 2004) and BnLIP2 of *Brassica napus* (21.5%, AAX59709) (Ling et al., 2006).

### 2.2. Spatio-temporal expression of tomato GDSL1

### 2.2.1. GDSL genes show a spatial-specific expression in vegetative tissues

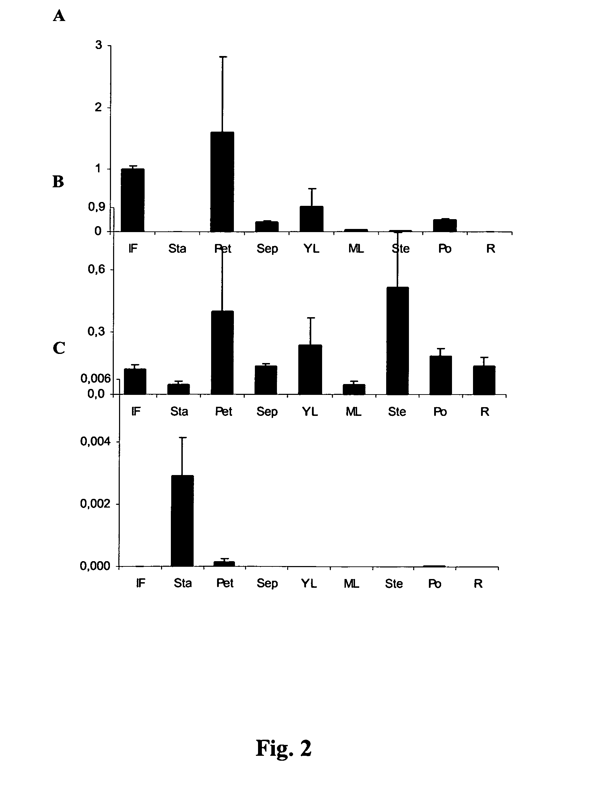

To investigate whether GDSL genes are expressed ubiquitously in tomato plants, quantitative RT-PCR was performed in various organs of wild-type plants: fruit at 20 DPA, inflorescences, anther, petal, sepal, young leaf, mature leaf, stem, hair, and root (figure 2). GDSL genes show important variation of expression levels. The mean transcript levels ranged in the order gdsl1 > gdsl2 > gdsl3. Furthermore, each gdsl gene seems to be specifically expressed in one or several tissues and totally absent of others. GDSL1 transcripts are highly detected in petal, fruit, stem and young leaf, but not in anther and root (figure 2A). GDSL2 is expressed in all tissues and more especially in petal and stem (figure 2B). GDSL3 is slightly expressed and specifically in anther and petal (figure 2C).

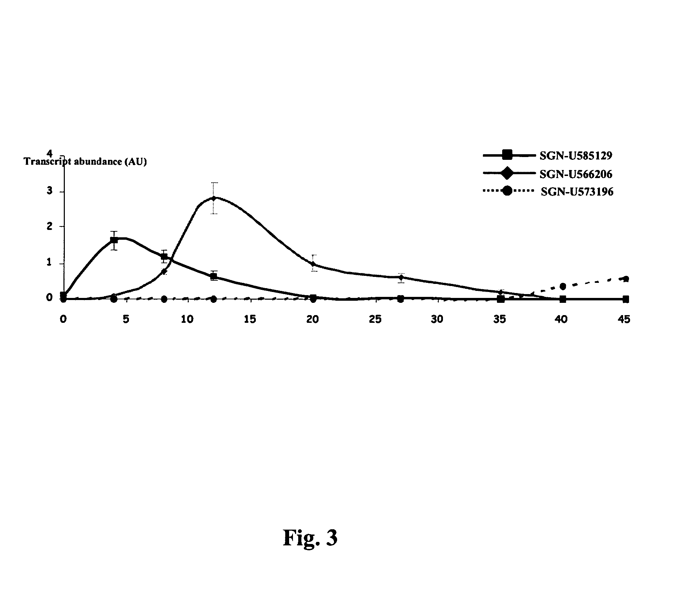

2.2.2. GDSL genes show a developmental stage-dependant expression Furthermore, the temporal expression of GDSL was investigated during fruit development was performed in whole fruit. RNAs were purified from fruit samples collected at 4, 8, 12, 20, 27, 35, 40 and 45 DPA and used as template to detect GDSL transcript level by quantitative RT-PCR analysis (figure 3). GDSL1 and GDSL2 genes were expressed during early tomato development (4 to 35 DPA) whereas gdsl3 was preferentially expressed during fruit ripening (35 - 45 DPA). gdsl1 transcripts showed a peak of expression at 12 DPA and decreased slowly until the end of fruit development. The expression of GDSL2 decreased along fruit development whereas whose of gdsl3 increased at later stage. *gdsl2* was the most precious: its highest level of expression was at 4DPA (days post anthesis) and decreased rapidly to nearly no expression as soon as 20DPA.

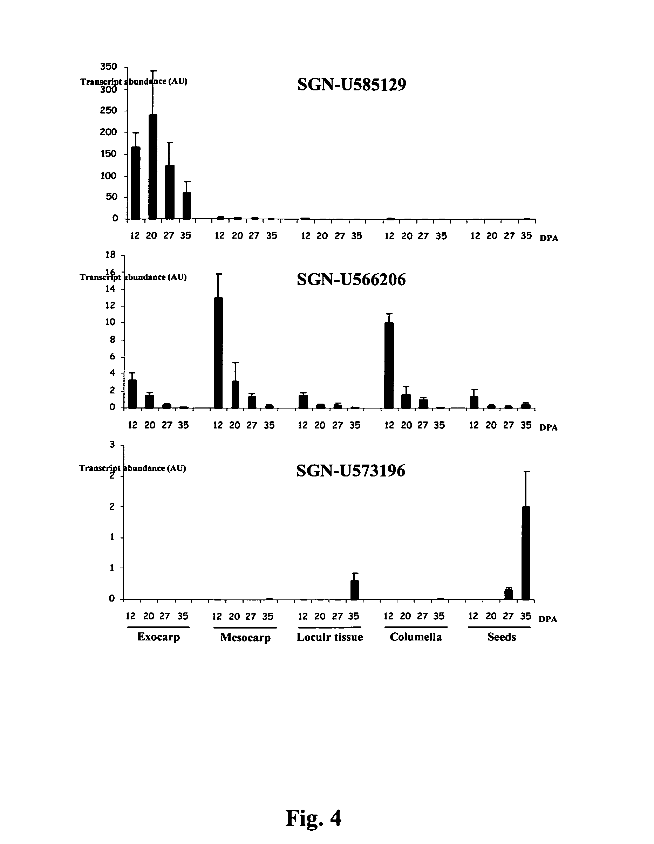

2.2.3. GDSL genes show a tissue-specific expression in tomato fruit Finally, expression of GDSL genes in fruit during its development was investigated in 5 different fruit tissues: seed, locular tissue, columella, mesocarp and exocarp (figure 4). Along fruit development, *gdsl* genes also presented specific expression pattern

Each GDSL gene shows a proper expression pattern (figure 4). gdsl1 displays the highest expression level among the gdsl tested, as already observed in whole fruit. Moreover, gdsl1 transcripts are specifically detected in exocarp. These transcripts are detected in this tissue at the four developmental stages tested, with a maximum of expression at 12 DPA. gdsl2 is expressed in all fruit tissues at early developmental stages. Its expression is more important in columella and exocarp at 12 DPA. gdsl3 transcripts are detected in seed and to a lower level in locular tissue at late developmental stages.

The enrichment of each tissue during this analysis revealed that GDSL1 highest expression is not at 12DPA as previously seen (figure 4) but more around 20DPA in exocarp. Expression rate repartition of GDSL2 is exocarp > columella > mesocarp > locular tissue > seeds and as expected, decreased during fruit development. Finally, SIGDSL3 expression is more important in seeds of 35DPA fruits, expression in locular tissue probably being a contamination by seeds during sampling.

2.3. Subcellular immunolocalisation of GDSL1 protein in tomato exocarp To investigate GDSL1 subcellular localization and to confirm its secretion in extracellular matrix, immunolocalisation was conducted on 20 DPA wt tomato exocarp.

In electron micrographs realized on WT samples, the cuticle of exocarp was seen as an electron dense layer of 5.4 to 21 µm deposited over a less dense layer corresponding to cell walls as previously observed in Ailsa Craig tomato at red ripe stage (Saladié et al 2007). TEM analysis of cuticles from wt fruits showed that cuticle penetrates between epidermis cells, forming an anticlinal peg corresponding to the beginning of the outer cell wall cutinisation.

Immunogold electron microscopy revealed that GDSL1 was clearly localized in cuticle as well as in the underlying epidermis cells whereas no labelling was observed in cell wall layer. This result demonstrated that GDSL1 is excreted from the underlying cells to the extracellular cuticle.

### 2.4. Alteration of GDSL1 lipase expression by RNAi in tomato

### 2.4.1. Generation of gdsl1-RNAi plants

Two RNAi constructions were generated, one using a 3'-UTR fragment and the other a 5' fragment as inverted repeat under control of 35S promoter. Ten T0 plants were selected: 3.1, 3.6, 4, 10, 12, 14, 17, 18 and 20 for 5'-RNAi construct and 7.2 for 3'-RNAi construct. The analysis of gdsl1 gene expression allows to quantify gdsl1 gene extinction and to select the most interesting plants.

### 2.4.2. Modification of gdsl genes expression in gdsl1 silenced plants

To investigate gdsl1 extinction efficiency, the expression of gdsl1 in leaves and flowers of different silenced plants in comparison to wild-type plant was performed by quantitative RT-PCR. Different levels of expression decrease were observed in the transgenic plants. In leaves, all transformed plants presented a decrease in gdsl1 relative expression rate, except plant 3.6. And for plants 4, 14, 17 and 18 reduction raised 10 fold less than in wt leaves. In flowers, gdsl1 expression rate was strongly reduced in all transformed plants. 4, 12, 17, 18 and 20 transformed plants showed a relative gdsl expression rate 10 fold lower than the wt plant. Several plants seemed to be particularly affected with a residual expression level of gdsl1 of 10% in leaves and in flowers (4, 17 and 18).

In addition, the expression of gdsl2 and gdsl3 was measured in the same samples and showed an increase of expression rate of gdsl2 of several transformed plants in comparison of wild-type plant. In leaves, the gdsl2 expression was at least doubled in 3.1, 10 and 18 transformed plants and in flowers, gdsl2 presented an increased expression in plants 3.6, 7.2, 10, 12 and 18 (x 1.5 in plants 3.6, 7.2, 10 and 12). These observations suggested a compensation of the decrease in gdsl1 expression by gdsl2. Nevertheless, no direct correlation was observed between the decrease of gdsl1 expression and the increase of gdsl2 transcript rate measured in some plants. For example, transformed plant 4 showed a decrease of gdsl1 expression rate in leaf but no increase of gdsl2 transcripts. On the contrary, the expression of gdsl1 in flowers was only slightly diminished in plant 3.6 and gdsl2 transcript rate was highly increased. No modification in expression rates of gdsl3 was observed.

### 2.4.3. Expression of gdsl lipases in fruits

One phenotype observed on gdsl1 silenced plant was a modification of flower structure leading to a low fertilization rate in some transgenic lines (3.6, 7.2, 12, 18 and 20) resulting in the absence of fruits. Thus, expression rates of gdsl genes were only measured in wild-type plant and 3.1, 4, 10 and 17 transformed plants. An important reduction in gdsl1 expression rate was observed especially for plants 4 and 17, where the gdsl1 remaining expression was close to zero. Two and five fold decreases of gdsl1 expression were observed in fruits of transformed plants 3.1 and 10, respectively. In contrast with that was observed in leaves and flowers, in fruits, no increase of gdsl2 and gdsl3 expression rates was noticed. According to these observations, 3.1, 4, 10 and 17 transformed plants were chosen to produce T2 homozygous plants which were used for biophysic studies.

### 2.5. Morphological and cuticular defects associated with the loss of GDSL1 function

### 2.5.1. Effects of gdsl1 gene silencing on plants

Influence of silencing of gdsl1 gene on cuticle structure was analyzed in T2 homozygous plants. Modifications of fruit colour at maturity and reduction of the number of fruits per truss was observed, but the major phenotype was the higher cuticle transparency and brightness on transformed plants. The use of hydrophilic toluidine blue dye (TB) coloration and the conservation tests allowed to suggest cuticle defects in 17.3 plants, and to less extend in 3.1.9 plants. In addition, tomato fruits were harvested at the red ripe growth stage of wild-type and transformed plants before any sign of dehydration and were stored at room temperature in order to assess their resistance to dehydration. After 3 weeks, only the 17.3 plant displayed visible sign of dehydration. Taken together, these results suggest that the fruit cuticle of the transformed plant is affected.

### 2.5.2. Determination of cuticle thickness in transformed plant

To investigate the influence of gdsl1 silencing on cuticle thickness, light microscopy observations were performed on 10 µm cross-sections of cuticle of mature fruits from T2 transformed and wild-type plant. As previously observed in red ripe tomato fruit (Bargel et al 2005; Buda et al 2009; Isaacson 2009) cuticles penetrates between epidermis cells, forming an anticlinal peg. Cuticle thickness was measured (i) at the cuticle membrane and (ii) between two epidermal cells including cuticle membrane and the anticlinal peg (figure 5).

Sharp differences were observed between Wt and the GDSL transformants particularly at the cuticle membrane level were the thickness reduction is correlated to the reduction of GDSL expression. (see Figure 5)

### 2.5.3. Morphological characterization of cuticle and cutin surface and cross section

Cuticle surface of mature tomato cuticle from wt and 17.3 transformant plant were compared by SEM. In the case of wild type plant, cuticle exhibited a regular morphology as previously described in the literature (Hovav, 2007). By contrast, cuticle surface from 17.3 transformant plant seemed less regular and wrinkled. Cuticle and cutin surface morphology has also been investigated through SEM and AFM at 20 DPA. The SEM images showed a continuous cuticle displaying the imprint of the underlying epidermis cells. The impression of the underlying plant cell of the cuticle is more important for the 17.3 plant as the design of the corresponding cuticle cells are more apparent in the corresponding SEM images as compared with the wt fruit cuticle. Similar observation was obtained from the AFM images that have been performed from isolated dewaxed cuticle samples. As a consequence, the 17.3 phenotype can be attributed to a lower cutin thickness; which is not compensated by wax deposition. As previously observed (Round et al., 2000), wild type cutin surface displayed regular domains whereas cuticle from the 17.3 transformant showed a more irregular texture.

Furthermore, high resolution AFM images of the corresponding cutin surfaces (wt and 17.3) display notable differences in the surface microstructures.The RMS roughness value has been estimated to 10.4 nm and 7.4 nm for respectively the wild type and the 17.3 transformant. While the roughness values are quite similar, significant difference can be seen from the corresponding morphologies. The fine surface of the wild sample can be described as an assembly of regular small regions ranging from 20 to 80 nm whereas the surface of the 17.3 transformant is designed by larger and irregular nodules.

The co-ordinated use of AFM and SEM indicated surface differences that may be involved in the brightness differences observed between 17.3 and wt tomato fruits.

In addition, nanopores with a mean diameter of 20 nm were observed in the topography of the 17.3 cutin and were absent in the wild type cutin, whereas the same sample preparation procedure was used for both cutin. These surface features are confirmed in each case by the AFM height images, assuming identical recording conditions for both wild and 17.3 samples tomato fruit.

These nanopores could account for the differences in the dehydration rate observed between Wild type and 17.3 tomato fruit.

### 2.5.4. ATR FTIR

FTIR was performed on cuticle and cutin isolated from wild plant and T2 transformant plants (4.2, 3.1.9, 10.10, 17.3). ATR spectroscopy is an effective technique for studying biological material because sample can be oriented on the ATR crystal. As a matter of fact, FTIR spectra were obtained from the external side and from the internal or "cell wall" side of cuticle (figures 6 and 7). From the external side, the spectra display typical cutin spectra dominated by aliphatic CH2 asymmetric and symmetric stretching bands at 2919 cm-1 and 2850 cm-1. Cutin show also a major band at 1729 cm-1 with a shoulder at 1708 cm-1 assigned to C=O stretching vibration of ester groups. The shoulder at 1708 cm-1 indicates that some of these functional group are involved in hydrogen bounding (Chen et al., 2005). The band at 1167 cm-1 is attributed to C-O ester bound. By contrast, spectra from the internal side of the cutin are dominated by a major polysaccharide feature at 1032 cm-1 (da Luz, 2006) and a characteristic broad OH bands in the 3360 cm-1 range. The obtained spectra were very similar between all plants except for the 17.3 transformant were the polysaccharide features are more important (as compared to the C=O moieties) than in the wild type. This may be explained as a result of a thinner cutin layer. According to the C=O stretching vibration, the same shoulder at 1708 cm-1 was observed for all plants, which suggest that the hydrogen bounding in the cuticle are not modified through gdsl1 silencing although a slight shift (2cm-1) was observed in the case of 17.3 plant.

### 2.5.5. Cutin composition

The cutin fraction isolated density from the different transformant and wild-type plant at the red ripe stage was 0.418 ± 0.08 mg/cm-2 for 17.3, 0.856 ± 0.002 mg/cm-2 for 3.1.9, 1.589 ± 0.21 mg/cm-2 for 4.2 and 1.03 ± 0.13 mg/cm-2 for 10.10 transformed plants in comparison to 1,336 ± 0.24 mg/cm-2 for wt plant. According to the literature (Baker et al., 1982; Osman et al., 1995) tomato cutin comprises 16-hydroxyhexadecanoic acid and dihydroxyhexadecanoic acids. It must be noticed however, that a significant amount of dicarboxylic acids, usually described as suberin monomers were also extracted from tomato cuticle. A similar result was reported from red tomato (Wang et al., 2000). Data are the average of three replications and variability for the cutin composition was high.

Nevertheless, according to this study, there were no significant differences in the relative composition (%) of cutin constituents for the different tested plants, except for a smaller (p<0.05) proportion of 16-hydroxyhexadecanoic acid.

### 2.6. In vitro activity of GDSL1 recombinant

2.6.1. Transformed Escherichia coli express hydrolase activity on tributyrin agar Observation of tributyrin agar plates after 48 h incubation gives a rapid obviousness of hydrolytic activity of bacterial culture. The diffusion test in tributyrin agar showed clear precipitate zones for E. coli expressed Thr-GDSL1 but not in E. coli transformed by pETD vector without insert. The depth of clear precipitate zone around wells increases during incubation time.

### 2.6.2. Recombinant GDSL1 (rGDSL1) show esterase activity toward substrates with short chain lengths

Hydrolytic activities of rGDSL1 and Thr-GDSL1 were measured using various *p*nitrophenyl derivatives with different acyl group chain lengths: *p*-nitrophenyl acetate (C2), *p*-nitrophenyl butyrate (C4), *p*-nitrophenyl laurate (C12), *p*-nitrophenyl myristolate (C14), and *p*-nitrophenyl palmitate (C16). Chain length of acyl groups allow discriminate esterase and lipase activities of hydrolytic enzymes. Enzymes shown lipase activity were characterized by their ability to hydrolyze a wide range of fatty acid esters whereas enzymes with esterase activity hydrolyze preferentially short chain fatty acid esters (Pringle and Dickstein, 2004; Hong et al., 2008). We found a hydrolase activity for rGLDS1 and Thr-GDSL1 using *p*-nitrophenyl acetate (C2), p-nitrophenyl butyrate (C4). No significant hydrolytic activity is detected when longer chains of pnitrophenyl esters were used as substrate.

### 3. Discussion

GDSL-motif lipase-acylhydrolase forms a large multigenic family and in the smallest plant genome sequenced, Arabidopsis thaliana 107 non redundant members have been recovered from data mining (Ling, 2008). The tetrapeptide motif G-D-S-L, the signature of this family of proteins is generally located close to the N-terminal region of the amino acid sequence (Akoh et al., 2004; Ling, 2008). The serine residue contained in this motif is involved in the hydrolase activity and differs from common lipases where the catalytic serine residue belong to a GxSxG motif located in the middle regions of their amino acid sequences (Akoh et al., 2004). No tri-dimensional structure is available for a plant member of this protein family. Only the crystal structure of a bacterial GDSL esterase has been solved and has been used as template for determining by homology the residues involved in the hydrolase activity and to highlight the consensus polypeptide blocks that contain the residues involved in catalysis and binding of substrates (Lo et al., 2003).

Interestingly, this unique structure showed that GDSL-motif lipase-acylhydrolases belong to the large family α/β hydrolase (Lo et al., 2003). Here, the GDSL lipase isolated and characterized from tomato fruit belongs obviously to this protein family and the different conserved blocks can be highlighted in its amino acid sequence. Although these proteins become more and more attractive in the recent years, their function is still unknown. The GDSL-motif lipase-acylhydrolases characterized so far have been related to plant defense (JPN1 (Kram et al., 2008), CaGL1 (Kim et al., 2008), Glip1 (Oh et al., 2005), AtLTL1 (Naranjo et al., 2006)), germination (Ling et al., 2006; Clauss et al., 2008) and morphogenesis (Ling et al., 2006). In the present study, it was clearly demonstrated that GDSL-motif lipase-acylhydrolases are also involved in the formation of plant cuticle.

All the data obtained show that the GDSL-motif lipase-acylhydrolase, GDSL1, is involved in the formation of the cutin polyester. Several lines of evidence support these hypotheses.

First, GDSL was an abundant protein in mature tomato cuticle whereas gdsl1 expression reaches its maximum at 12 DPA. Moreover, GDSL1 was secreted in the cell wall apoplast as verified by expression of YFP-GDSL1 fusion protein and in agreement with the presence of a signal peptide at the N-terminus. Extracellular localization is quite a common feature of the GDSLs described so far (Oh et al., 2005; Hong et al., 2008; Kim et al., 2008). Finally (iii) in tomato fruit, 32 unigenes expressed in fruit are presented in databank (SOL genomics network). Numerous copies of the same family genes suggest an important role in plant development. A high efficiency in gdsl1 silencing demonstrates its importance in cutin formation. Nevertheless, silencing of gdsl1 expression might be compensated by the expression of other GDSL genes. For instance, even if its expression level is low, gdsl2 is expressed in the exocarp at the early stage of fruit development. As a consequence, GDSL2 production might be sufficient to maintain a minimal cutin formation along the fruit development.

With regard to the function of GDSL1, *in vitro* analysis of the recombinant protein revealed an esterase activity. Esterase activity has also been described for other GDSLs.Enod8, a GDSL isolated from *Medicago sativa* nodules (Pringle and Dickstein, 2004) and associated to suberin formation exhibited in vitro esterase activity. Recently, BnSCE3 isolated from *Brassica napus* and involved in the early stage of seed germination showed a sinapine esterase activity (Clauss et al., 2008). On the contrary, delineation of the cuticular phenotype of transformed plant suggests that the protein is closely related to the polymerization of the cutin. A double function including both esterase/hydrolase and transferase activities has already been demonstrated for other lipases like lipolytic enzyme of *Aeromonas hydrophila* (Upton and Buckley, 1995).

The GDSL transformant exhibited a sharp reduction of cuticle thickness. In addition, severe deformation of epidermal cell wall was not observed. These results contrast with the cuticle of BODYGUARD mutants. Bodyguard encodes a protein candidate for cutin polymerization and/or anchoring the lipid polymers to the cell wall. Actually, Kurduyov and coworkers (Kurdyukov et al., 2006) observed an irregular cutin accumulation (above and within the cell wall) associated with the accumulation of cutin and wax in the *Arabidopsis* plants interrupted in the BODYGUARD gene. Interestingly, BODYGUARD encodes also an esterase with the α/β hydrolase fold. However, in regard to the GDSL-motif lipase-acylhydrolases, this esterase belongs to a different family of proteins. As a consequence, GDSL-motif lipase-acylhydrolases and α/β hydrolases, the two candidates for cutin polymerization are differently regulated and are involved in different mecanisms.

### REFERENCES

Akoh, C.C., Lee, G.-C., Liaw, Y.-C., Huang, T.-H., and Shaw, J.-F. (2004). GDSL family of serine esterases/lipases. Progress in Lipid Research 43, 534-552.
Baker, E.A., Bukova C, M.J., and Hunt, G.M. (1982). Composition of tomato fruit cuticle as related to fruit growth and development. In The plant cuticle, D.F. Cutler, K.L. Alvin, and C.E. Price, eds (New York: academic press), pp. 33-44.
Bargel, H., and Neinhuis, C. (2005). Tomato (Lycopersicon esculentum Mill.) fruit growth and ripening as related to the biomechanical properties of fruit skin and isolated cuticle. Journal of Experimental Botany 56, 1049-1060.
Beisson, F., Li, Y.H., Bonaventure, G., Pollard, M., and Ohlrogge, J.B. (2007). The acyltransferase GPAT5 is required for the synthesis of suberin in seed coat and root of Arabidopsis. The Plant Cell 19, 351-368.
Bessire, M., Chassot, C., Jacquat, A.-C., Humphry, M., Borel, S., MacDonald-Comber Petétot, J., Métraux, J.-P., and Nawrath, C. (2007). A permeable cuticle in Arabidopsis leads to a strong resistance to Botrytis cinerea. Embo Journal 26, 2158-2168.
Bird, D.A. (2008). The role of ABC transporters in cuticular lipid secretion. Plant Science 174, 563-569.
Blee, E., and Schuber, F. (1993). Biosynthesis of cutin monomers: involvement of a lipoxygenase/peroxygenase pathway. The Plant Journal 4**.**
Chen, B., Zhu, L., Zhu, J., and Xing, B. (2005). Configurations of the Bentonite-Sorbed Myristylpyridinium Cation and Their Influences on the Uptake of Organic Compounds. Environmental Science and Technology 39, 6093-6100.
Clauss, K., Baumert, A., Nimtz, M., Milkowski, C., and Dieter Strack. (2008). Role of a GDSL lipase-like protein as sinapine esterase in Brassicaceae. The Plant Journal 53, 802-813.
da Luz, B.R. (2006). Attenuated total reflectance spectroscopy of plant leaves: a tool for ecological and botanical studies. New Phytologist 172, 305-318.
Gilbert, R.D., Johnson, A.M., and Dean, R.A. (1996). Chemical signals responsible for appressorium formation in the rice blast fungus Magnaporthe grisea. Physiological and Molecular Plant Pathology 48, 335-346.
Graça, J., Schreiber, L., Rodrigues, J., and Pereira, H. (2002). Glycerol and glyceryl esters of omega-hydroxyacids in cutins. Phytochemistry 61, 205-215. Hamza, S., and Chupeau, Y. (1993). Re-evaluation of Conditions for Plant Regeneration and Agrobacterium-Mediated Transformation from Tomato (Lycopersicon esculentum). Journal of Experimental Botany 44, 1837-1845.
Hong, J., Choi, H., Hwang, I., Kim, D., Kim, N., Choi, D., Kim, Y., and Hwang, B. (2008). Function of a novel GDSL-type pepper lipase gene, CaGLIP1, in disease susceptibility and abiotic stress tolerance. Planta 227, 539-558.
Hovav, R., Chehanovsky, N., Moy, M., Jetter, R., and Schaffer, A.A. (2007). The identification of a gene (Cwp1), silenced during Solanum evolution, which causes cuticle microfissuring and dehydration when expressed in tomato fruit. The Plant Journal 52, 627-639.
Iwamoto, M., Takeuchi, Y., Takada, Y., and Yamaoka, N. (2002). Coleoptile surface cuticle of barley is involved in survival and penetration of Blumeria graminis. Physiological and Molecular Plant Pathology 60, 31-38.
Kannangara, R., Branigan, C., Liu, Y., Penfield, T., Rao, V., Mouille, G., Hofte, H., Pauly, M., Riechmann, J.L., and Broun, P. (2007). The Transcription Factor WIN1/SHN1 Regulates Cutin Biosynthesis in Arabidopsis thaliana. The Plant Cell 19, 1278-1294.
Karimi, M., Inzé, D., and Depicker, A. (2002). GATEWAY(TM) vectors for Agrobacteriummediated plant transformation. Trends in Plant Science 7, 193-195. Kersteins, G. (1996). Plant cuticles: an integrated approach. (oxford: BIOS Scientific Publisherd Limited).
Kim, K.-J., Lim, J.H., Kim, M.J., Kim, T., Chung, H.M., and Paek, K.-H. (2008). GDSLlipase1 (CaGL1) contributes to wound stress resistance by modulation of CaPR-4 expression in hot pepper. Biochemical and Biophysical Research Communications 374, 693-698.
Kolattuduky, P. (1996). Biosynthetic pathways of cutin and waxes, and their sensitivity to environmental stresses. In Plant cuticles: an integrated approach, G. Kersteins, ed (Oxford: BIOS Scientific Publishers Limited), pp. 83-118.
Kram, B., Bainbridge, E., Perera, M., and Carter, C. (2008). Identification, cloning and characterization of a GDSL lipase secreted into the nectar of Jacaranda mimosifolia. Plant Molecular Biology 68, 173-183.
Kurdyukov, S., Faust, A., Nawrath, C., Bar, S., Voisin, D., Efremova, N., Franke, R., Schreiber, L., Saedler, H., Metraux, J.-P., and Yephremov, A. (2006). The Epidermis-Specific Extracellular BODYGUARD Controls Cuticle Development and Morphogenesis in Arabidopsis. The Plant Cell 18, 321-339.
Lemaire-Chamley, M., Petit, J., Garcia, V., Just, D., Baldet, P., Germain, V., Fagard, M., Mouassite, M., Cheniclet, C., and Rothan, C. (2005). Changes in Transcriptional Profiles Are Associated with Early Fruit Tissue Specialization in Tomato. Plant Physiology 139, 750-769.
Li, Y., Fred, B., Abraham, J.K.K., Isabel, M., Mike, P., and John, O. (2007). Identification of acyltransferases required for cutin biosynthesis and production of cutin with suberin-like monomers. Proceedings of the National Academy of Sciences of the USA 104, 18339-18344.
Ling, H. (2008). Sequence analysis of GDSL lipase gene family in Arabidopsis thaliana. Pakistan Journal of Biological Sciences 11, 763-767.
Ling, H., Zhao, J., Zuo, K., Qiu, C., Yao, H., Qin, J., Sun, X., and Tang, K. (2006). Isolation and expression analysis of a GDSL-like lipase gene from Brassica napus L. Journal of Biochemistry and Molecular Biology 39, 297-303.
Lo, Y.-C., Lin, S.-C., Shaw, J.-F., and Liaw, Y.-C. (2003). Crystal Structure of Escherichia coli Thioesterase Protease Lysophospholipase L1: Consensus Sequence Blocks Constitute the Catalytic Center of SGNH-hydrolases through a Conserved Hydrogen Bond Network. Journal of Molecular Biology 330, 539-551.
Naranjo, M.A., Forment, J., Roldan, M., Serrano, R., and Vicente, O. (2006). Overexpression of Arabidopsis thaliana LTL1, a salt-induced gene encoding a GDSLmotif lipase, increases salt tolerance in yeast and transgenic plants. Plant, Cell and Environment 29, 1890-1900.
Nawrath, C. (2006). Unraveling the complex network of cuticular structure and function. Current Opinion in Plant Biology Physiology and metabolism 9, 281-287. Oh, I., Park, A., Bae, M., Kwon, S., Kim, Y., Lee, J., Kang, N., Lee, S., Cheong, H., and Park, O.K. (2005). Secretome analysis reveals an Arabidopsis lipase involved in defense against Alternaria brassicicola. The Plant Cell 17, 2832-2847. Osman, S.F., Gerard, H.C., Fett, W.F., Moreau, R.A., and Dudley, R.L. (1995). Method for the production and characterization of tomato cutin oligomers. Journal of Agricultural and Food Chemistry 43, 2134-2137.
Panikashvili, D., Savaidi-Goldstein, S., Mandel, T., Yifhar, T., Franke, R.B., Hofer, R., Schreiber, L., Chory, J., and Aharoni, A. (2007). The Arabidopsis DESPERADO/AtWBC11 Transporter Is Required for Cutin and Wax Secretion. Plant Physiology 145, 1345-1360.
Petracek, P.D., and Bukovac, M.J. (1995). Rheological Properties of Enzymatically Isolated Tomato Fruit Cuticle. Plant Physiology 109, 675-679.
Pollard, M., Beisson, F., Li, Y., and Ohlrogge, J.B. (2008). Building lipid barriers: biosynthesis of cutin and suberin. Trends in Plant Science 13, 236-246.
Pringle, D., and Dickstein, R. (2004). Purification of ENOD8 proteins from Medicago sativa root nodules and their characterization as esterases. Plant Physiology and Biochemistry 42, 73-79.
Pyee, J., Yu, H.S., and Kolattukudy, P.E. (1994). Identification of a Lipid Transfer Protein as the Major Protein in the Surface Wax of Broccoli (Brassica oleracea) Leaves. Archives of Biochemistry and Biophysics 311, 460-468.
Rashid, N., Shimada, Y., Ezaki, S., Atomi, H., and lmanaka, T. (2001). Low-Temperature Lipase from Psychrotrophic Pseudomonas sp. Strain KB700A. Applied and Environmental Microbiology 67, 4064-4069.
Reina, J.J., Guerrero, C., and Heredia, A. (2007). Isolation, characterization, and localization of AgaSGNH cDNA: a new SGNH-motif plant hydrolase specific to Agave americana L. leaf epidermis. Journal of Experimental Botany 58, 2717-2731. Riederer, M., and Schreiber, L. (2001). Protecting against water loss: analysis of the barrier properties of plant cuticles. Journal of Experimental Botany 52, 2023-2032.
Round, A.N., Yan, B., Dang, S., Estephan, R., Stark, R.E., and Batteas, J.D. (2000). The Influence of Water on the Nanomechanical Behavior of the Plant Biopolyester Cutin as Studied by AFM and Solid-State NMR. Biophysical Journal 79, 2761-2767.
Ruiz, C., Falcocchio, S., Xoxi, E., Javier Pastor, F.I., Diaz, P., and Saso, L. (2004). Activation and inhibition of Candida rugosa and Bacillus-related lipases by saturated fatty acids, evaluated by a new colorimetric microassay. Biochimica and Biophysica Acta (BBA) - General Subjects 1672, 184-191.
Schnurr, J., Shockey, J., and Browse, J. (2004). The Acyl-CoA Synthetase Encoded by LACS2 Is Essential for Normal Cuticle Development in Arabidopsis. The Plant Cell 16, 629-642.
Schweizer, P., Felix, G., Buchala, A., Muller, C., and Metraux, J.P. (1996). Perception of free cutin monomers by plant cells. The Plant Journal 10, 331-341. Shieh, M.W., Wessler, S.R., and Raikhel, N.V. (1993). Nuclear Targeting of the Maize R Protein Requires Two Nuclear Localization Sequences. Plant Physiology 101, 353-361.
Sieber, P., Schorderet, M., Ryser, U., Buchala, A., Kolattukudy, P., Metraux, J.-P.,and Nawrath, C. (2000). Transgenic Arabidopsis Plants Expressing a Fungal Cutinase Show Alterations in the Structure and Properties of the Cuticle and Postgenital Organ Fusions. The Plant Cell 12, 721-738.
Sterk, P., Booij, H., Schellekens, G.A., Van Kammen, A., and De Vries, S.C. (1991). Cell-specific expression of the carrot EP2 lipid transfer protein gene. The Plant Cell 3, 907-921.
Suh, M.C., Samuels, A.L., Jetter, R., Kunst, L., Pollard, M., Ohlrogge, J., and Beisson, F. (2005). Cuticular Lipid Composition, Surface Structure, and Gene Expression in Arabidopsis Stem Epidermis. Plant Physiology 139, 1649-1665.
Taketa, S., Amano, S., Tsujino, Y., Sato, T., Saisho, D., Kakeda, K., Nomura, M., Suzuki, T., Matsumoto, T., Sato, K., Kanamori, H., Kawasaki, S., and Takeda, K. (2008). Barley grain with adhering hulls is controlled by an ERF family transcription factor gene regulating a lipid biosynthesis pathway. Proceedings of the National Academy of Sciences of the USA 105, 4062-4067.
Tanaka, T., Tanaka, H., Machida, C., Watanabe, M., and Machida, Y. (2004). A new method for rapid visualization of defects in leaf cuticle reveals five intrinsic patterns of surface defects in Arabidopsis. The Plant Journal 37, 139-146.
Upton, C., and Buckley, J.T. (1995). A new family of lipolytic enzymes? Trends in Biochemical Sciences 20, 178-179.
Waltson, T. (1990). Waxes, cutin and suberin. In Lipids, membranes and aspects of photobiology, J. Harwood and J. Boyer, eds (London: Academic Press), pp. 105-158.
Wang, C., Chin, C., and Gianfagna, T. (2000). Relationship between cutin monomers and tomato resistance to powdery mildew infection. Physiological and Molecular Plant Pathology 57.
Wellesen, K., Durst, F., Pinot, F., Benveniste, I., Nettesheim, K., Wisman, E. , Steiner-Lange, S., Saedler, H., and Yephremov, A. (2001). Functional analysis of the LACERATA gene of Arabidopsis provides evidence for different roles of fatty acid omega-hydroxylation in development. Proceedings of the National Academy of Sciences of the USA 98, 9694-9699.
Xiao, F., Goodwin, S.M., Xiao, Y., Sun, Z., Baker, D., Tang, X., Jenks, M.A., and Zhou, J.M. (2004). Arabidopsis CYP86A2 represses Pseudomonas syringae type III genes and is required for cuticle development. Embo Journal 23, 2903-2913.
McCallum et al, 2000 Nat Biotechnol. 18(4):455-7; reviewed by Stemple, 2004, Nat Rev Genet. 5(2): 145-50.
Colbert et al., 2001, Plant Physiol. 126(2):480-4
Slade et al., 2005, Nat Biotechnol. 23(1):75-81
Lightner and Caspar, 1998, "Seed mutagenesis of Arabidopsis", In: Methods in Molecular Biology: Arabidopsis Protocols (eds. J. M. Martinez-Zapater and J. Salinas), pp. 91-103. Totowa, New Jersey: Humana Press)
Draper et al., 2004, Methods Cell Biol. 2004;77:91-I 12
Buda GJ, Isaacson T, Matas AJ, Paolillo DJ, Rose JK. Three-dimensional imaging of lant cuticle architecture using confocal scanning laser microscopy.
Isaacson T, Kosma DK, Matas AJ, Buda GJ, He Y, Yu B, Pravitasari A, Batteas JD, Stark RE, Jenks MA, Rose JK. Cutin deficiency in the tomato fruit cuticle consistently affects resistance to microbial infection and biomechanical properties, but not transpirational water loss.

## Claims

1. An isolated plant GDSL-lipase polypeptide differentially expressed between the different steps of the plant development, preferably of the fruit development and/or the different tissues of the plant, preferably of the fruit, comprising the sequences SEQ ID NO:1, SEQ NO:2, SEQ ID NO:3, SEQ ID NO:4 and SEQ ID NO:5 in this order from the N-terminal to the C-terminal ends

2. An isolated plant GDSL-lipase polypeptide according to claim 1, differentially expressed between seed, locular tissue, columella, mesocarp and exocarp of the fruit.

3. An isolated plant GDSL-lipase polypeptide according to claim 1 or 2, specifically expressed in the exocarp of the fruit.

4. An isolated plant GDSL-lipase polypeptide according to any of the preceding claims comprising the sequences SEQ ID NO:1, SEQ NO:2, SEQ ID NO:3, SEQ ID NO:4 and SEQ ID NO:5 in this order from the N-terminal to the C-terminal ends separated by sequences which present at least 60%, preferably at least 70%, and more preferably at least 80% with the sequence SEQ ID NO:6, SEQ ID NO:7 or SEQ ID NO:8 its orthologs, and derivatives thereof.

5. An isolated plant GDSL-lipase polypeptide according to any of claims 1 to 3 comprising
(a) a polypeptide sequence selected in the group consisting of SEQ ID NO:6, SEQ ID NO:7 or SEQ ID NO:8, or
(b) a polypeptide sequence which is at least 60%, preferably at least 70%, and more preferably at least 80%, the most preferably at least 90% identical to the polypeptide of (a), said polypeptide being differentially expressed between the different steps of the plant development, preferably of the fruit development and/or the different tissues of the plant, preferably of the fruit, or
(c) a polypeptide sequence being a fragment of any one of (a) to (b), wherein said fragment or biologically active portion thereof is differentially expressed between the different steps of the plant development, preferably of the fruit development and/or the different tissues of the plant, preferably of the fruit.

6. An isolated plant GDSL-lipase polypeptide according to any of the preceding claims comprising the sequence SEQ ID NO:6, SEQ ID NO:7 or SEQ ID NO:8.

7. An isolated plant GDSL-lipase polypeptide according to any of the preceding claims, wherein said sequence is a tomato sequence, a hot pepper sequence or a sweet pepper sequence.

8. An isolated polynucleotide comprising a nucleic acid molecule encoding for a polypeptide as defined in any of the previous claims.

9. The polynucleotide of claim 8, comprising a sequence selected from the group consisting of SEQ ID NO:9, SEQ ID NO:10 or SEQ ID NO:11.

10. A vector comprising the polynucleotide as defined in claim 8 or 9.

11. A host cell genetically engineered with the polynucleotide as defined in claim 8 or 9, or with the vector defined in claim 10.

12. A monoclonal antibody directed to a polypeptide as claimed in claims 1 to 7.

13. A plant or part of plant genetically engineered to not express at least partially an isolated plant GDSL-lipase polypeptide according to any of claims 1 to 7.

14. A tomato plant or a part of a tomato plant genetically engineered to not express at least partially an isolated plant GDSL-lipase polypeptide selected from the group consisting of GDSL1 (SEQ ID NO:6), GDSL2 (SEQ ID NO:7) or GDSL3 (SEQ ID NO:8).

15. A tomato fruit genetically engineered to not express at least partially GDSL1 (SEQ ID NO:6).

16. A plant or part of plant genetically engineered to express an isolated plant GDSL-lipase polypeptide according to any of claims 1 to 7 having an abnormal activity.

17. A plant, part of plant or fruit according to any one of claims 12 to 15 which is transgenic.

18. A plant, part of plant or fruit according to claim 17 comprising a knock-out of a polynucleotide according to claim 8 or 9.

19. A method for obtaining a plant mutant carrying at least a point mutation which suppresses at least partially the expression of an isolated plant GDSL-lipase polypeptide according to any of claims 1 to 6, said method comprising the following steps:
a. A mutagenesis treatment of seeds with a chemical mutagen such as Ethyl methanesulfonate (EMS);
b. Self-fertilization of the resulting M1 plants;
c. Extraction of DNA samples of the M2 individuals for mutational screening;
d. The DNA samples are pooled and arrayed in microtiter plates, and the pools are amplified using primers specific of a polypeptide as claimed in claims 1 to 6;
e. Amplification products are incubated with the CEL I or ENDO1 endonuclease;
f. Cleavage products are electrophoresed to identify punctual mutations in said polypeptide.

20. Use of a part of plant according to any one of claims 13 to 18 or of a part of a plant mutant obtainable by the method of claim 19 which is a fruit for preparing puree, juice or coulis of said fruit.

21. Use of a part of plant according to any one of claims 13 to 18 or a part of a plant mutant obtainable by the method of claim 19 for preparing an extract, preferably without using organic solvents.

22. Use of a tomato fruit genetically engineered to not express at least partially an isolated plant GDSL-lipase polypeptide according to any one of claims 1 to 7 or a tomato fruit carrying at least a point mutation obtainable by the method according to claim 19 for preparing canned peeled tomatoes, tomato juice, tomato paste or tomato ketchup.


SEQUENCE LISTING

<110>  Institut National de la Recherche Agronomique (INRA)

<120>  Isolated plant GDSL lipase

<130>  67994

<160>  50

<170>  PatentIn version 3.3

<210>  1
<211>  9
<212>  PRT
<213>  artificial

<220>
<223>

<220>
<221>  MISC_FEATURE
<222>  (1)..(1)
<223>  X is F or L

<220>
<221>  misc_feature
<222>  (6)..(6)
<223>  Xaa can be any naturally occurring amino acid

<220>
<221>  misc_feature
<222>  (8)..(8)
<223>  Xaa can be any naturally occurring amino acid

<400>  1

Xaa Gly Asp Ser Leu Xaa Asp Xaa Gly
1               5

<210>  2
<211>  12
<212>  PRT
<213>  artificial

<220>
<223>

<220>
<221>  misc_feature
<222>  (4)..(4)
<223>  X is F or C or Y

<220>
<221>  misc_feature
<222>  (5)..(5)
<223>  X is S or C

<220>
<221>  misc_feature
<222>  (6)..(6)
<223>  X is N or D

<220>
<221>  misc_feature
<222>  (8)..(8)

<223>  X is any amino acid

<220>
<221>  misc_feature
<222>  (9)..(9)
<223>  X is any amino acid

<220>
<221>  misc_feature
<222>  (10)..(10)
<223>  X is any amino acid

<220>
<221>  misc_feature
<222>  (11)..(11)
<223>  X is any amino acid

<400>  2

Thr Gly Arg Xaa Xaa Xaa Gly Xaa Xaa Xaa Xaa Asp
1               5                   10


<210>  3
<211>  4
<212>  PRT
<213>  artificial

<220>
<223>



<220>
<221>  misc_feature
<222>  (2)..(2)
<223>  X is G or S

<220>
<221>  misc_feature
<222>  (3)..(3)
<223>  X is N or D

<220>
<221>  misc_feature
<222>  (4)..(4)
<223>  X is D or E

<400>  3

Gly Xaa Xaa Xaa
1


<210>  4
<211>  18
<212>  PRT
<213>  artificial

<220>
<223>



<220>
<221>  misc_feature
<222>  (1)..(1)
<223>  X is any amino acid

<220>
<221>  misc_feature

<222>  (4)..(4)
<223>  X is any amino acid

<220>
<221>  misc_feature
<222>  (6)..(6)
<223>  X is V or I

<220>
<221>  misc_feature
<222>  (7)..(7)
<223>  X is any amino acid

<220>
<221>  misc_feature
<222>  (8)..(8)
<223>  X is any amino acid

<220>
<221>  misc_feature
<222>  (9)..(9)
<223>  X is any amino acid

<220>
<221>  misc_feature
<222>  (10)..(10)
<223>  X is any amino acid

<220>
<221>  misc_feature
<222>  (11)..(11)
<223>  X is any amino acid

<220>
<221>  misc_feature
<222>  (12)..(12)
<223>  X is any amino acid

<220>
<221>  misc_feature
<222>  (14)..(14)
<223>  X is any amino acid

<220>
<221>  misc_feature
<222>  (17)..(17)
<223>  X is any amino acid

<400>  4

Xaa Gly Ala Xaa Arg Xaa Xaa Val Xaa Xaa Xaa Xaa Pro Xaa Gly Cys
1               5                   10                  15

Xaa Pro

<210>  5
<211>  16
<212>  PRT
<213>  artificial

<220>
<223>

<220>
<221>  misc_feature

<222>  (2)..(2)
<223>  X is any amino acid

<220>
<221>  misc_feature
<222>  (3)..(3)
<223>  X is N or D

<220>
<221>  misc_feature
<222>  (4)..(9)
<223>  X is any amino acid

<220>
<221>  misc_feature
<222>  (12)..(13)
<223>  X is any amino acid

<220>
<221>  misc_feature
<222>  (15)..(15)
<223>  X is P or L

<220>
<221>  misc_feature
<222>  (16)..(16)
<223>  X is S or T

<400>  5

Cys Xaa Xaa Xaa Xaa Xaa Xaa Xaa Xaa Trp Asp Xaa Xaa His Xaa Xaa
1               5                   10                  15


<210>  6
<211>  362
<212>  PRT
<213>  Solanum lycopersicum

<400>  6

Met Ala Thr Pro Thr Ile Ile Leu Ser Phe Leu Leu Ile Phe Gly Val
1               5                   10                  15


Ala Ile Cys Gln Ser Glu Ala Arg Ala Phe Phe Val Phe Gly Asp Ser
            20                  25                  30


Leu Val Asp Ser Gly Asn Asn Asn Tyr Leu Ala Thr Thr Ala Arg Ala
        35                  40                  45


Asp Ser Pro Pro Tyr Gly Ile Asp Tyr Pro Thr Arg Arg Ala Thr Gly
    50                  55                  60


Arg Phe Ser Asn Gly Tyr Asn Ile Pro Asp Ile Ile Ser Gln Gln Ile
65                  70                  75                  80


Gly Ser Ser Glu Ser Pro Leu Pro Tyr Leu Asp Pro Ala Leu Thr Gly
                85                  90                  95


Gln Arg Leu Leu Val Gly Ala Asn Phe Ala Ser Ala Gly Ile Gly Ile
            100                 105                 110

Leu Asn Asp Thr Gly Ile Gln Phe Ile Asn Ile Ile Arg Met Pro Gln
        115                 120                 125

Gln Leu Ala Tyr Phe Arg Gln Tyr Gln Ser Arg Val Ser Gly Leu Ile
    130                 135                 140

Gly Glu Ala Asn Thr Gln Arg Leu Val Asn Gln Ala Leu Val Leu Met
145                 150                 155                 160

Thr Leu Gly Gly Asn Asp Phe Val Asn Asn Tyr Tyr Leu Val Pro Asn
                165                 170                 175

Ser Ala Arg Ser Arg Gln Phe Ser Ile Gln Asp Tyr Val Pro Tyr Leu
            180                 185                 190

Ile Arg Glu Tyr Arg Lys Ile Leu Met Asn Val Tyr Asn Leu Gly Ala
        195                 200                 205

Arg Arg Val Ile Val Thr Gly Thr Gly Pro Leu Gly Cys Val Pro Ala
    210                 215                 220

Glu Leu Ala Gln Arg Ser Arg Asn Gly Glu Cys Ser Pro Glu Leu Gln
225                 230                 235                 240

Arg Ala Ala Gly Leu Phe Asn Pro Gln Leu Thr Gln Met Leu Gln Gly
                245                 250                 255

Leu Asn Ser Glu Leu Gly Ser Asp Val Phe Ile Ala Ala Asn Thr Gln
            260                 265                 270

Gln Met His Thr Asn Phe Ile Thr Asn Pro Gln Ala Tyr Gly Phe Ile
        275                 280                 285

Thr Ser Lys Val Ala Cys Cys Gly Gln Gly Pro Tyr Asn Gly Leu Gly
    290                 295                 300

Leu Cys Thr Pro Leu Ser Asn Leu Cys Pro Asn Arg Asp Val Tyr Ala
305                 310                 315                 320

Phe Trp Asp Pro Phe His Pro Ser Glu Arg Ala Asn Lys Ile Ile Val
                325                 330                 335

Gln Gln Ile Met Ser Gly Thr Thr Glu Leu Met Asn Pro Met Asn Leu
            340                 345                 350

Ser Thr Ile Leu Ala Met Asp Ser His Ala
        355                 360

<210>  7
<211>  369
<212>  PRT
<213>  Solanum lycopersicum

<400>  7

Met Ala Thr Phe Ser Leu Thr Lys Val Ser Leu Ser Val Leu Leu Val
1               5                   10                  15

Ser Leu Phe Phe Phe Ser Pro Ser Ser Ala Gln Thr Lys Cys Asn Val
            20                  25                  30

Asn Ser Val Tyr Gln Leu Gly Asp Ser Leu Ala Asp Ala Gly Asn Val
        35                  40                  45

Ile Arg Thr Pro Gly Ala Ser Ile Ile Phe Arg Ala Asp Arg Ser Pro
    50                  55                  60

Tyr Gly Glu Thr Phe Phe Arg Lys Pro Thr Gly Arg Phe Ser Asn Gly
65                  70                  75                  80

Arg Val Ile Thr Asp Phe Ile Ser Gln Ser Phe Lys Leu Pro Phe Leu
                85                  90                  95

Asn Ala Tyr Leu Asp Arg Thr Ala Ser Phe Ser Gln Gly Val Asn Phe
            100                 105                 110

Ala Val Ala Gly Ala Thr Ala Leu Asp Thr Ser Phe Trp Thr Ala Arg
        115                 120                 125

Asn Ile Arg Leu Pro Thr Trp Asn Thr Pro Leu Ser Asn Gln Leu Thr
    130                 135                 140

Trp Phe Lys Ser His Leu Lys Ser Thr Cys Gly Ser Lys Cys Ala Glu
145                 150                 155                 160

Asn Leu Lys Asn Ser Ile Val Ile Met Gly Glu Trp Gly Gly Asn Asp
                165                 170                 175

Tyr Tyr Asn Cys Phe Phe Gln Arg Lys Gln Ile Ser Glu Val Arg Ser
            180                 185                 190

Tyr Val Pro Phe Val Val Ala Gly Ile Met Arg Gly Ile Lys Glu Val
        195                 200                 205

Ile Gln Leu Gly Ala Thr Arg Val Leu Val Pro Ser Ile Tyr Pro Leu
    210                 215                 220

Gly Cys Leu Pro Leu Tyr Leu Thr Ser Phe Pro Asp Asn Asn Thr Ser
225                 230                 235                 240

Gly Tyr Asp Gln Leu Gly Cys Leu Lys Ser Phe Asn Asp Phe Ala Ser
                245                 250                 255

Tyr His Asn Arg Phe Val Val Arg Ala Met Ala Asn Leu Gln Arg Gln
            260                 265                 270

Phe Pro Asn Val Ser Ile Val Tyr Gly Asp Phe Tyr Gly Ser Ile Leu
        275                 280                 285

Ser Leu Ile Arg Asn Pro Ser Ser Phe Gly Phe Asn Gln Asn Thr Leu
    290                 295                 300

Leu Ser Ala Cys Cys Gly Thr Gly Gly Thr His Asn Phe Asn Phe Arg
305                 310                 315                 320

Thr Val Cys Gly Gly Ala Gly Ile Asn Ala Cys Ser Asn Pro Ala Lys
                325                 330                 335

Tyr Val His Trp Asp Gly Ile His Leu Thr Asp Glu Ala His Arg Arg
            340                 345                 350

Ile Ser Glu Val Leu Val Arg Asp Met Leu Ala Lys Phe Asn Cys Val
        355                 360                 365

Val

<210>  8
<211>  380
<212>  PRT
<213>  Solanum lycopersicum

<400>  8

Met Ala Phe Thr Ile Arg Val Ala Val Leu His Leu Leu Ile Leu Glu
1               5                   10                  15

Ile Thr Ile Ile Ser Ser Leu Phe Leu Gln Ala Asn Ala Gln Glu Leu
            20                  25                  30

Leu Ile Lys Leu Glu Glu Pro Lys Leu Lys Lys Cys Gly Ile Asn Arg
        35                  40                  45

Ile Tyr Gln Phe Gly Asp Ser Leu Ser Asp Thr Gly Asn Cys Leu Arg
    50                  55                  60

Glu Ser Tyr Cys Gly Ala His Thr Gly Cys Arg Phe Pro Pro Tyr Gly
65                  70                  75                  80

Met Asn Phe Tyr Gln Lys Glu Ile Thr Gly Arg Cys Ser Asn Gly Leu
                85                  90                  95

Leu Met Val Asp Phe Met Ala Leu Glu Cys Gly Leu Pro Leu Leu Asn
            100                 105                 110

Pro Ser Leu Glu Glu Asn Ala Asn Phe Ser Gln Gly Ser Asn Phe Ala
        115                 120                 125

Val Ala Gly Ala Thr Ala Leu Ser Val Glu Gln Leu Ala Glu Arg Asn

130                 135                 140

Ile Ser Met Ser Tyr Thr Asn Ser Thr Leu Thr Val Gln Leu His Trp
145                 150                 155                 160

Met Ser Ser His Phe Asn Ser Ile Cys Ser Thr Asp Cys Pro Lys Tyr
                165                 170                 175

Leu Glu Lys Ser Leu Phe Leu Val Gly Glu Ile Gly Gly Asp Glu Leu
            180                 185                 190

Ala Tyr Gly Phe Ile Gln Gly Lys Thr Ile Gln Glu Leu Arg Thr Met
        195                 200                 205

Val Pro Asp Ile Val His Ala Ile Ile Arg Gly Val Thr Arg Val Ile
    210                 215                 220

Ser Phe Gly Ala Thr Arg Ile Val Val Pro Gly Asn Ile Pro Ala Gly
225                 230                 235                 240

Cys Asn Pro Val Ile Leu Thr Ile Phe Gly Thr Thr Tyr Asp Glu Tyr
                245                 250                 255

Gly Cys Val Lys Glu Trp Asn Asn Phe Met Met Tyr Tyr Asn Asn His
            260                 265                 270

Leu Lys Arg Gly Val Tyr Met Leu Lys Arg Glu Tyr Pro Asn Ile Ser
        275                 280                 285

Ile Ser Tyr Gly Asp Tyr Tyr Asn Ala Tyr Leu Trp Leu Arg Gln Asn
    290                 295                 300

Val Val Ala Leu Gly Phe Asp Lys Asn Ser Val Leu Lys Ala Cys Cys
305                 310                 315                 320

Gly Ile Gly Gly Glu Tyr Asn Tyr Arg Ala Asp Met Arg Cys Gly Asn
                325                 330                 335

Pro Gly Val Lys Ala Cys Ala Asp Pro Asn Ser Tyr Ile Ser Trp Asp
            340                 345                 350

Gly Ile His Leu Thr Gln Arg Ala Tyr Ser Trp Leu Thr Lys Trp Leu
        355                 360                 365

Ile Asp Asp Ile Ile Pro Gln Leu Asn Cys Leu Val
    370                 375                 380

<210>  9
<211>  9
<212>  PRT
<213>  artificial

<220>

<223>

<400>  9

Phe Gly Asp Ser Leu Val Asp Ser Gly
1               5


<210>  10
<211>  12
<212>  PRT
<213>  artificial

<220>
<223>


<220>
<221>  misc_feature
<222>  (4)..(4)
<223>  X is F or C

<220>
<221>  misc_feature
<222>  (8)..(9)
<223>  X is any amino acid

<220>
<221>  misc_feature
<222>  (10)..(10)
<223>  X is I or H

<220>
<221>  misc_feature
<222>  (11)..(11)
<223>  X is any amino acid

<400>  10

Thr Gly Arg Xaa Ser Asn Gly Xaa Xaa Xaa Xaa Asp
1               5                   10


<210>  11
<211>  7
<212>  PRT
<213>  artificial

<220>
<223>


<220>
<221>  misc_feature
<222>  (5)..(6)
<223>  X is any amino acid

<400>  11

Gly Gly Asn Asp Xaa Xaa Asn
1               5


<210>  12
<211>  18
<212>  PRT
<213>  artificial

<220>

<223>

<220>
<221>  misc_feature
<222>  (4)..(4)
<223>  X is any amino acid

<220>
<221>  misc_feature
<222>  (7)..(12)
<223>  X is any amino acid

<220>
<221>  misc_feature
<222>  (17)..(17)
<223>  X is any amino acid

<400>  12

Leu Gly Ala Xaa Arg Val Xaa Val Xaa Xaa Xaa Xaa Pro Leu Gly Cys
1               5                   10                  15

Xaa Pro

<210>  13
<211>  20
<212>  PRT
<213>  artificial

<220>
<223>

<220>
<221>  misc_feature
<222>  (6)..(7)
<223>  X is any amino acid

<220>
<221>  misc_feature
<222>  (13)..(13)
<223>  X is any amino acid

<220>
<221>  misc_feature
<222>  (17)..(17)
<223>  X is any amino acid

<220>
<221>  misc_feature
<222>  (19)..(19)
<223>  X is any amino acid

<400>  13

Leu Cys Pro Asn Arg Xaa Xaa Tyr Ala Phe Trp Asp Xaa Phe His Pro
1               5                   10                  15

Xaa Glu Xaa Ala
            20

<210>  14
<211>  12

<212>  PRT
<213>  artificial

<220>
<223>



<220>
<221>  misc_feature
<222>  (8)..(8)
<223>  X is any amino acid

<220>
<221>  misc_feature
<222>  (11)..(11)
<223>  X is any amino acid

<400>  14

Thr Gly Arg Phe Ser Asn Gly Xaa Asn Ile Xaa Asp
1               5                   10


<210>  15
<211>  13
<212>  PRT
<213>  artificial

<220>
<223>



<220>
<221>  misc_feature
<222>  (4)..(5)
<223>  X is any amino acid

<220>
<221>  misc_feature
<222>  (11)..(12)
<223>  X is any amino acid

<400>  15

Leu Val Leu Xaa Xaa Leu Gly Gly Asn Asp Xaa Xaa Asn
1               5                   10


<210>  16
<211>  16
<212>  PRT
<213>  artificial

<220>
<223>



<220>
<221>  misc_feature
<222>  (5)..(5)
<223>  X is any amino acid

<220>
<221>  misc_feature
<222>  (7)..(7)
<223>  X is any amino acid

<400>  16

Leu Gly Ala Arg Xaa Val Xaa Val Thr Gly Thr Gly Pro Leu Gly Cys
1               5                   10                  15


<210>  17
<211>  20
<212>  PRT
<213>  artificial

<220>
<223>



<220>
<221>  misc_feature
<222>  (4)..(4)
<223>  X is any amino acid

<220>
<221>  misc_feature
<222>  (9)..(9)
<223>  X is any amino acid

<220>
<221>  misc_feature
<222>  (11)..(11)
<223>  X is any amino acid

<400>  17

Leu Val Asn Xaa Ala Leu Val Leu Xaa Phe Xaa Gly Gly Asn Asp Phe
1               5                   10                  15


Val Asn Asn Tyr
            20


<210>  18
<211>  3
<212>  PRT
<213>  artificial

<220>
<223>

<400>  18

Pro Tyr Gly
1


<210>  19
<211>  12
<212>  PRT
<213>  artificial

<220>
<223>


<220>
<221>  misc_feature
<222>  (2)..(2)
<223>  X is any amino acid

<220>
<221>  misc_feature
<222>  (4)..(4)

<223>  X is F or Y

<220>
<221>  misc_feature
<222>  (6)..(6)
<223>  X is S or V

<220>
<221>  misc_feature
<222>  (8)..(8)
<223>  X is G or A

<220>
<221>  misc_feature
<222>  (9)..(11)
<223>  X is any amino acid

<400>  19

Gly Xaa Asn Xaa Ala Xaa Ala Xaa Xaa Xaa Xaa Leu
1               5                   10


<210>  20
<211>  12
<212>  PRT
<213>  artificial

<220>
<223>


<220>
<221>  misc_feature
<222>  (2)..(2)
<223>  X is any amino acid

<400>  20

Gly Xaa Asn Phe Ala Ser Ala Gly Ile Gly Ile Leu
1               5                   10


<210>  21
<211>  12
<212>  PRT
<213>  artificial

<220>
<223>


<220>
<221>  misc_feature
<222>  (2)..(2)
<223>  X is any amino acid

<400>  21

Gly Xaa Asn Phe Ala Val Ala Gly Ala Thr Ala Leu
1               5                   10


<210>  22
<211>  6
<212>  PRT
<213>  artificial

<220>

<223>

<220>
<221>  misc_feature
<222>  (5)..(5)
<223>  X is any amino acid

<400>  22

Ala Cys Cys Gly Xaa Gly
1               5

<210>  23
<211>  8
<212>  PRT
<213>  artificial

<220>
<223>

<220>
<221>  misc_feature
<222>  (4)..(4)
<223>  X is any amino acid

<400>  23

Arg Ala Asp Xaa Pro Pro Tyr Gly
1               5

<210>  24
<211>  1432
<212>  DNA
<213>  Solanum lycopersicum

<400>  24
ttggcggcca atcggccgaa ttgaaatgca caaaccaatt tataatattt ttgtaacaac      60

acactttttt ttattttttt tgccaaaatg gccacaccta ctattatttt gagcttcttg     120

ttgatttttg gagtggctat ttgtcaaagt gaagctaggg cattttttgt gtttggtgat     180

tcacttgttg atagtggaaa taataattat ttggctacta ctgcaagggc tgattcacca     240

ccttatggta ttgattatcc aacacgtaga gcaactggtc gtttctctaa tggctacaac     300

attcctgaca ttatcagtca acaaattggt tcatcagagt caccactacc ttacttagat     360

ccagctctta ctggacaaag acttcttgtt ggtgctaact ttgcatctgc tggaattgga     420

atactaaatg acactggaat ccaatttatt aatattattc gaatgccaca acaattggct     480

tattttagac aatatcaaag tagagtaagt ggccttattg gtgaagcaaa tactcaaaga     540

cttgtaaatc aagctcttgt tcttatgact cttggaggca atgattttgt caacaactat     600

tatcttgtgc ccaattctgc gcgatcacgc caattttcta ttcaagatta tgtcccttat     660

ttgataagag aatatcgtaa aatcttgatg aatgtgtata atcttggagc tcgtcgtgta     720

attgtaactg gaactggacc gttaggttgt gttccagcag aactagctca acgtagcagg     780

aacggggaat gttcacccga gttgcaacga gctgcaggcc tgtttaaccc ccagcttacg     840

caaatgttgc aggggttaaa tagtgaacta ggcagcgatg tttttattgc tgcaaataca     900

caacaaatgc atacgaattt cattactaat ccacaagcat atggatttat aacatcaaag 960

gtagcatgtt gtggacaagg accatataac ggtcttggtc tatgtacacc gctctctaat 1020

ttgtgcccga atagagatgt ttacgcgttt tgggacccgt tccatccatc tgagagggca 1080

aataagatca ttgtgcagca aatcatgtct ggtacaacgg agcttatgaa tccaatgaat 1140

ctcagtacga ttctggctat ggattcacat gcataagaca tatctaagat atctggaatc 1200

tgattcactt gtaccttttt tgttgctaat ttttggctat aaataagatg tatgcaacac 1260

ttcatgttgt gctactttta aatttacaaa aaagtttggt tgtgtgctat gtttttattc 1320

acataattca gtaatctaat ttgtagggtg gagtgtgata ttgttgaaga tgtaaaccaa 1380

agtgttttta ttaatttata tagtaatata ttttcagtgt ttaaaaaaaa aa 1432

<210> 25
<211> 1110
<212> DNA
<213> Solanum lycopersicum

<400> 25
atggcgactt tttctcttac caaagtctcc ttatccgttt tactcgtctc tttattcttc 60

ttttctcctt catctgctca aactaaatgc aacgttaact ccgtttacca actcggcgac 120

tccctcgccg acgccggaaa tgtcatccgt actcccggcg cttccatcat cttccgcgcc 180

gaccgttccc cttacggcga aactttcttc agaaaaccca ccggccgctt ctccaacggc 240

cgcgttatca ccgatttcat ttctcaatca ttcaaacttc cattcctcaa cgcttactta 300

gacagaaccg catcgttcag tcaaggagta aacttcgccg tcgccggagc taccgccctc 360

gatacatcct tctggactgc acgtaacatc cgtttaccca cctggaacac accgctttca 420

aatcaattaa cttggttcaa atcgcatctg aaatcgactt gtggatcgaa atgtgcagag 480

aatttgaaaa actcgatcgt aataatgggg gaatggggag gaaacgatta ctacaattgt 540

ttcttccaga gaaagcaaat ctccgaagtc agaagttacg ttccgtttgt tgttgcaggg 600

ataatgagag gcatcaaaga ggtgattcag ctgggagcaa cacgagtgtt ggttccgagc 660

atttaccctc tcggatgcct tccattatac ctcacatcct tccctgataa caatacaagt 720

ggttacgatc aattaggatg cctgaaaagc ttcaatgatt tcgcttccta tcacaatcga 780

ttcgtggtta gagctatggc caatctacaa cgtcaatttc caaatgttag tattgtttat 840

ggtgattttt atggatcaat tttgtcactt attcgtaatc cgtcttcgtt tgggtttaat 900

caaaatacat tgctgagtgc atgttgtgga actggaggaa cacataattt caacttcagg 960

actgtatgtg gaggagctgg gattaatgct tgttcgaatc cagcaaagta tgttcattgg 1020

gacggaattc atttgacaga tgaagctcat cgtcgtattt ctgaagttct ggttagagat 1080

atgttagcca aattcaactg cgtcgtttaa 1110

<210> 26
<211> 1818
<212> DNA
<213> Solanum lycopersicum

<400>  26
aggattcggc acgagggcat tcacaataag agttgcggtg cttcatctgt tgatactaga      60

aataacaata ataagttcgt tgtttcttca agctaacgca caagaattat taataaagct     120

cgaagaacca aaattgaaga aatgcggaat taatagaatt tatcagtttg gtgattcact     180

ttccgatacc ggaaattgcc tgagagagag ctattgtgga gctcatactg ggtgtagatt     240

ccctccttat ggaatgaatt tttaccagaa agaaataacg gggcgttgtt ctaatggatt     300

gctcatggtt gatttcatgg cgctggaatg tggtcttcct ctcctaaatc cgtccttaga     360

agaaaatgca aattttagtc agggttcgaa ttttgcagta gcaggggcta cggctttatc     420

agttgaacaa cttgcagaaa ggaacatttc tatgtcttat acaaatagta cattaactgt     480

gcagcttcat tggatgtctt ctcatttcaa ttccatttgc tctacggatt gcccaaaata     540

tttggaaaag tcacttttcc tagttggaga aataggagga gatgaacttg cgtatgggtt     600

tatccaaggt aaaaccatac aagagttgcg aacaatggtg cctgacattg ttcatgccat     660

tattcgcggt gttacatggc attcacaata agagttgcgg tgcttcatct gttgatacta     720

gaaataacaa taataagttc gttgtttctt caagctaacg cacaagaatt attaataaag     780

ctcgaagaac caaaattgaa gaaatgcgga attaatagaa tttatcagtt tggtgattca     840

ctttccgata ccggaaattg cctgagagag agctattgtg gagctcatac tgggtgtaga     900

ttccctcctt atggaatgaa tttttaccag aaagaaataa cggggcgttg ttctaatgga     960

ttgctcatgg ttgatttcat ggcgctggaa tgtggtcttc ctctcctaaa tccgtcctta    1020

gaagaaaatg caaattttag tcagggttcg aattttgcag tagcaggggc tacggcttta    1080

tcagttgaac aacttgcaga aaggaacatt tctatgtctt atacaaatag tacattaact    1140

gtgcagcttc attggatgtc ttctcatttc aattccattt gctctacgga ttgcccaaaa    1200

tatttggaaa agtcactttt cctagttgga gaaataggag gagatgaact tgcgtatggg    1260

tttatccaag gtaaaaccat acaagagttg cgaacaatgg tgcctgacat tgttcatgcc    1320

attattcgcg gtgttacaag agttataagt tttggagcta ctcgaattgt agttccaggt    1380

aatattccag ctggttgtaa cccggttatc ctgacgatat ttggtaccac atacgatgag    1440

tatggttgcg tcaaagagtg gaacaatttt atgatgtatt acaacaatca tttgaaacga    1500

ggagtttaca tgctcaagag agaatatcca aacatttcaa tcagttacgg cgactactac    1560

aacgcttatc tgtggcttcg acaaaatgtt gttgctcttg gattcgacaa aaattctgta    1620

ctgaaagcat gttgtggaat aggtggagag tacaattata gagcagatat gagatgtggt    1680

aatccaggag tcaaagcgtg tgcagacccc aatagttaca taagttggga tggaattcat    1740

ttaacacaaa gggcatacag ttggttaaca aaatggctaa tcgatgacat aataccacaa    1800

ttaaactgcc ttgtttga                                                  1818

<210>  27
<211>  20
<212>  DNA
<213>  artificial

<220>
<223>  Primer GDSL1 3'

<400>  27
taaagcttat gcatgtgaat                                                    20


<210>  28
<211>  31
<212>  DNA
<213>  artificial

<220>
<223>  GDSL1 5'

<400>  28
atccatgggg caaagtgaag ctagggcatt t                                       31


<210>  29
<211>  16
<212>  PRT
<213>  Artificial

<220>
<223>

<400>  29

Arg Ala Phe Phe Val Phe Gly Asp Ser Leu Val Asp Ser Gly Asn Asn
1               5                   10                  15


<210>  30
<211>  8
<212>  PRT
<213>  Artificial

<220>
<223>

<400>  30

Gly Gly Asn Asp Phe Val Asn Asn
1               5


<210>  31
<211>  32
<212>  DNA
<213>  Artificial

<220>
<223>  GDSL1s1 primer

<400>  31
aaaaagcagg ctccaaaatg gccacaccta ct                                      32


<210>  32
<211>  35
<212>  DNA
<213>  Artificial

<220>
<223>  GDSL1s2 primer

<400>  32

aaaaagcagg cttttttgtt gctaattttt gccta                                35

<210>  33
<211>  33
<212>  DNA
<213>  Artificial

<220>
<223>  GDSL1as3 primer

<400>  33
agaaagctgg gttgacaaaa atcattgcct cca                                  33

<210>  34
<211>  36
<212>  DNA
<213>  Artificial

<220>
<223>  GDSL1as4 primer

<400>  34
agaaagctgg gtcaacaata tccacactcc acccta                               36

<210>  35
<211>  31
<212>  DNA
<213>  Artificial

<220>
<223>  attB1 primer

<220>
<221>  misc_feature
<222>  (30)..(31)
<223>  n is a, c, g, or t

<400>  35
ggggacaagt ttgtacaaaa aagcaggctn n                                    31

<210>  36
<211>  31
<212>  DNA
<213>  Artificial

<220>
<223>  attB2 primer

<220>
<221>  misc_feature
<222>  (30)..(31)
<223>  n is a, c, g, or t

<400>  36
ggggaccact ttgtacaaga aagctgggtn n                                    31

<210>  37
<211>  24
<212>  DNA
<213>  Artificial

<220>

<223>  gdsl1 forward primer

<400>  37
gcaacacttc atgttgtgct actt                                              24


<210>  38
<211>  24
<212>  DNA
<213>  Artificial

<220>
<223>  gdsl1 reverse primer

<400>  38
caatatcaca ctccacccta caaa                                              24


<210>  39
<211>  22
<212>  DNA
<213>  Artificial

<220>
<223>  gdsl2 forward primer

<400>  39
gccaaattca actgcgtcgt tt                                                22


<210>  40
<211>  22
<212>  DNA
<213>  Artificial

<220>
<223>  gdsl2 reverse primer

<400>  40
tctccttcgc tacaacgtgg aa                                                22


<210>  41
<211>  20
<212>  DNA
<213>  Artificial

<220>
<223>  gdsl3 forward primer

<400>  41
gggtggccaa ttacaaaatg                                                   20


<210>  42
<211>  25
<212>  DNA
<213>  Artificial

<220>
<223>  gdsl3 reverse primer

<400>  42
actcacacac atcacataaa gatgc                                             25


<210>  43
<211>  20
<212>  DNA

<213>  Artificial

<220>
<223>  b-tubulin forward primer

<400>  43
aacctctcgt ggatcacagc                                                    20


<210>  44
<211>  21
<212>  DNA
<213>  Artificial

<220>
<223>  b-tubulin reverse primer

<400>  44
ggcagaagct gtcaggtaac g                                                  21


<210>  45
<211>  21
<212>  DNA
<213>  Artificial

<220>
<223>  EiF4a forward primer

<400>  45
agtggacgat ttggaaggaa g                                                  21


<210>  46
<211>  20
<212>  DNA
<213>  Artificial

<220>
<223>  EiF4a reverse primer

<400>  46
gctcctcgat tacgacgttg                                                    20


<210>  47
<211>  21
<212>  DNA
<213>  Artificial

<220>
<223>  actin forward primer

<400>  47
ggactctggt gatggtgtta g                                                  21


<210>  48
<211>  19
<212>  DNA
<213>  Artificial

<220>
<223>  actin reverse primer

<400>  48
ccgttcagca gtagtggtg                                                     19

<210>  49
<211>  510
<212>  DNA
<213>  Artificial

<220>
<223>

<400>  49
ccaaaatggc cacacctact attattttga gcttcttgtt gatttttgga gtggctattt      60

gtcaaagtga agctagggca ttttttgtgt ttggtgattc acttgttgat agtggaaata     120

ataattattt ggctactact gcaagggctg attcaccacc ttatggtatt gattatccaa     180

cacgtagagc aactggtcgt ttctctaatg gctacaacat tcctgacatt atcagtcaac     240

aaattggttc atcagagtca ccactacctt acttagatcc agctcttact ggacaaagac     300

ttcttgttgg tgctaacttt gcatctgctg gaattggaat actaaatgac actggaatcc     360

aatttattaa tattattcga atgccacaac aattggctta ttttagacaa tatcaaagta     420

gagtaagtgg ccttattggt gaagcaaata ctcaaagact tgtaaatcaa gctcttgttc     480

ttatgactct tggaggcaat gattttgtca                                       510


<210>  50
<211>  148
<212>  DNA
<213>  Artificial

<220>
<223>

<400>  50
ttttttgttg ctaatttttg gctataaata agatgtatgc aacacttcat gttgtgctac      60

ttttaaattt acaaaaaagt ttggttgtgt gctatgtttt tattcacata attcagtaat     120

ctaatttgta gggtggagtg tgatattg                                         148